# EUROPÄISCHE PATENTSCHRIFT

(11) **EP 1 799 649 B1**
(45) Veröffentlichungstag und Bekanntmachung des Hinweises auf die Patenterteilung: **25.06.2014**
(21) Anmeldenummer: 05787451.3
(22) Anmeldetag: 14.09.2005
(51) Int. Cl.: C07D 231/18, A61K 31/415, A61K 31/4155, C07D 401/12, C07D 403/12

(54) **3-(2-HYDROXYPHENYL)-PYRAZOLE UND IHRE VERWENDUNG ALS HSP90 MODULATOREN**
3-(2-HYDROXYPHENYL) PYRAZOLES AND THEIR USE AS HSP90 MODULATORS
3-(2-HYDROXYPHENYL)-PYRAZOLES ET LEUR UTILISATION EN TANT QUE MODULATEURS DE HSP90

(30) Priorität: 08.10.2004 DE 102004049078
(43) Veröffentlichungstag der Anmeldung: 27.06.2007
(73) Patentinhaber: Merck Patent GmbH, 64293 Darmstadt (DE)
(72) Erfinder: EGGENWEILER, Hans-Michael, 64291 Darmstadt (DE); WOLF, Michael, 64297 Darmstadt (DE)
(86) Internationale Anmeldenummer: PCT/EP2005/009873
(87) Internationale Veröffentlichungsnummer: WO 2006/039977

(56) Entgegenhaltungen:
- WO-A-03/055860
- WO-A-2004/050087
- ARKHIPOV, V. V. ET AL: "Chemistry of modified flavonoids. Part 24. Synthesis of 4- aryloxy-3-(2-hydroxy-4-hydroxy/alkoxypheny l)pyrazoles" CHEMISTRY OF HETEROCYCLIC COMPOUNDS (NEW YORK, NY, UNITED STATES)(TRANSLATION OF KHIMIYA GETEROTSIKLICHESKIKH SOEDINENII) , 40(2), 183-187 CODEN: CHCCAL; ISSN: 0009-3122, Februar 2004 (2004-02), XP002357317
- DATABASE REGISTRY [Online] CHEMICAL ABSTRACTS SERVICE, COLUMBUS, OHIO, US; RN 716333-47-0 26. Juli 2004 (2004-07-26), XP002357343 gefunden im STN -& DATABASE REGISTRY [Online] CHEMICAL ABSTRACTS SERVICE , COLUMBUS , OHIO, US; RN 716332-28-4 26. Juli 2004 (2004-07-26), XP002358298 gefunden im STN & DATABASE CHEMCATS STN; Chemical Library; Supplier: PHARMEKS Ltd.
- DATABASE CA [Online] CHEMICAL ABSTRACTS SERVICE, COLUMBUS, OHIO, US; TOMURA, ARIHIRO ET AL: "Preparation of pyrazoles as HSP90 inhibitors and their use as antitumor agents" XP002357344 gefunden im STN Database accession no. 2005:888155 -& JP 2005 225787 A (NIPPON KAYAKU CO., LTD., JAPAN) 25. August 2005 (2005-08-25)
- CHEUNG K M J ET AL: "The identification, synthesis, protein crystal structure and in vitro biochemical evaluation of a new 3,4-diarylpyrazole class of Hsp90 inhibitors" BIOORGANIC & MEDICINAL CHEMISTRY LETTERS, OXFORD, GB, Bd. 15, Nr. 14, 15. Juli 2005 (2005-07-15), Seiten 3338-3343, XP004944659 ISSN: 0960-894X

## Beschreibung

### INTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen, bei denen die Hemmung, Regulierung und/oder Modulation von HSP90 eine Rolle spielt, ferner pharmazeutische Zusammensetzungen, die diese Verbindungen enthalten, sowie die Verwendung der Verbindungen zur Behandlung von Krankheiten, bei denen HSP90 eine Rolle spielt.

Die korrekte Faltung und Konformation von Proteinen in Zellen wird durch molekulare Chaperone gewährleistet und ist kritisch für die Regulation des Gleichgewichts zwischen Protein Synthese und Degradation. Chaperone sind wichtig für die Regulation vieler zentraler Funktionen von Zellen wie z.B. Zellproliferation und Apoptose (Jolly and Morimoto, 2000; Smith et al., 1998; Smith, 2001).

### Hitzeschock-Proteine (heat shock proteins, HSPs)

Die Zellen eines Gewebes reagieren auf äußerlichen Stress wie z.B. Hitze, Hypoxie, oxidativem Stress, oder Giftstoffen wie Schwermetallen oder Alkoholen mit der Aktivierung einer Reihe von Chaperonen, welche unter der Bezeichnung "heat shock proteins" (HSPs) bekannt sind.

Die Aktivierung von HSPs schützt die Zelle gegen Verletzungen, die durch solche Stressfaktoren ausgelöst werden, beschleunigt die Wiederherstellung des physiologischen Zustands und führt zu einem stresstoleranten Zustand der Zelle.

Neben diesem ursprünglich entdeckten durch HSPs vermittelten Schutzmechanismus bei äußerlichem Stress wurden im Laufe der Zeit weitere wichtige Chaperon-Funktionen für einzelne HSPs auch unter normalen stressfreien Bedingungen beschrieben. So regulieren verschiedene HSPs beispielsweise die korrekte Faltung, die intrazelluläre Lokalisierung und Funktion oder den geregelten Abbau einer Reihe biologisch wichtiger Proteine von Zellen.

HSPs bilden eine Genfamilie mit individuellen Genprodukten, deren Zellulärexpression, Funktion und Lokalisierung in verschiedenen Zellen sich unterscheidet. Die Benennung und Einteilung innerhalb der Familie erfolgt aufgrund ihres Molekulargewichts z.B. HSP27, HSP70, and HSP90.

Einigen menschlichen Krankheiten liegt eine falsche Proteinfaltung zugrunde (siehe Review z.B. Tytell et al., 2001; Smith et al., 1998). Die Entwicklung von Therapien, welche in den Mechanismus der Chaperon abhängigen Proteinfaltung eingreift, könnte daher in solchen Fällen nützlich sein. Beispielsweise führen bei der Alzheimer-Erkrankung, Prionenerkrankungen oder dem Huntington Syndrom falsch gefaltete Proteine zu einer Aggregation von Protein mit neurodegenerativem Verlauf. Durch falsche Proteinfaltung kann auch ein Verlust der Wildtyp-Funktion entstehen, der eine fehlregulierte molekulare und physiologische Funktion zur Folge haben kann.

HSPs wird auch eine grosse Bedeutung bei Tumorerkrankungen beigemessen. Es gibt z.B. Hinweise, dass die Expression bestimmter HSPs im Zusammenhang mit dem Stadium der Progression von Tumoren steht (Martin et al., 2000; Conroy et al., 1996; Kawanishi et al., 1999; Jameel et al., 1992; Hoang et al., 2000; Lebeau et al., 1991).

Die Tatsache, dass HSP90 bei mehreren zentralen onkogenen Signalwegen in der Zelle eine Rolle spielt und gewisse Naturstoffe mit krebshemmender Aktivität HSP90 targetieren, führte zu dem Konzept, dass eine Hemmung der Funktion von HSP90 bei der Behandlung von Tumorerkrankungen sinnvoll wäre.

Ein HSP90 Inhibitor, 17-Allylamino-17-demethoxygeldanamycin (17AAG), ein Derivat von Geldanamycin, befindet sich gegenwärtig in klinischer Prüfung.

### HSP90

HSP90 repräsentiert ungefähr 1-2% der gesamten zellulären Proteinmasse. Es liegt in der Zelle gewöhnlich als Dimer vor und ist mit einer Vielzahl von Proteinen, sogenannten Co-chaperonen assoziiert (siehe z.B. Pratt, 1997). HSP90 ist essentiell für die Vitalität von Zellen (Young et al., 2001) und spielt eine Schlüsselrolle in der Antwort auf zellulären Stress durch Interaktion mit vielen Proteinen, deren native Faltung durch äußerlichen Stress, wie z.B. Hitzeschock, verändert wurde, um die ursprüngliche Faltung wiederherzustellen oder die Aggregation der Proteine zu verhindern (Smith et al., 1998).

Es gibt auch Hinweise, dass HSP90 als Puffer gegen die Auswirkungen von Mutationen eine Bedeutung hat, vermutlich durch die Korrektur falscher Proteinfaltung, die durch die Mutation hervorgerufen wurde (Rutherford and Lindquist, 1998).

Darüber hinaus hat HSP90 auch eine regulatorische Bedeutung. Unter physiologischen Bedingungen spielt HSP90, zusammen mit seinem Homolog im Endoplasmatischen Retikulum, GRP94, eine Rolle im Zellhaushalt, um die Stabilität der Konformation und Reifung verschiedener "client" Schlüsselproteine zu gewährleisten. Diese können in drei Gruppen unterteilt werden: Rezeptoren für Steroidhormone, Ser/Thr or Tyrosinkinasen (z.B. ERBB2, RAF-1, CDK4 und LCK) und einer Sammlung unterschiedlicher Proteine wie z.B. mutiertes p53 oder die katalytische Untereinheit der Telomerase hTERT. Jedes dieser Proteine nimmt eine Schlüsselrolle in der Regulation physiologischer und biochemischer Prozesse von Zellen ein.

Die konservierte HSP90-Familie des Menschen besteht aus vier Genen, dem zytosolischen HSP90α, der induzierbaren HSP90β Isoform (Hickey et al., 1989), dem GRP94 im Endoplasmatischen Retikulum (Argon et al., 1999) und dem HSP75/TRAP1 in der mitochondrialen Matrix (Felts et al., 2000). Es wird angenommen, dass alle Mitglieder der Familie eine ähnliche Wirkweise haben, aber, je nach ihrer Lokalisierung in der Zelle, an unterschiedliche "client" Proteine binden. Beispielsweise ist ERBB2 ein spezifisches "client" Protein von GRP94 (Argon et al., 1999), während der Typ1 Rezeptor des Tumornekrosefaktors (TNFR1) oder das Retinoblastom Protein (Rb) als "clients" von TRAP1 nachgewiesen wurden (Song et al., 1995; Chen et al., 1996).

HSP90 ist an einer Reihe von komplexen Interaktionen mit einer grossen Zahl von "client" Proteinen und regulatorischen Proteinen beteiligt (Smith, 2001). Obwohl präzise molekulare Details noch nicht geklärt sind, haben biochemische Experimente und Untersuchungen mit Hilfe der Röntgenkristallographie in den letzten Jahren zunehmend Details der Chaperonfunktion von HSP90 entschlüsseln können (Prodromou et al., 1997; Stebbins et al., 1997). Danach ist HSP90 ein ATP-abhängiges molekulares Chaperon (Prodromou et al, 1997), wobei die Dimerisierung wichtig für die ATP Hydrolyse ist. Die Bindung von ATP resultiert in der Formation einer toroidalen Dimerstruktur, bei der die beiden N-terminalen Domainen in engem Kontakt zueinander kommen und einen "switch" in der Konformation bewirken. (Prodromou and Pearl, 2000).

### Bekannte HSP90 Inhibitoren

Die erste Klasse von HSP90 Inhibitoren, die entdeckt wurde, waren Benzochinon-Ansamycine mit den Verbindungen Herbimycin A und Geldanamycin. Ursprünglich wurde mit ihnen die Reversion des malignen Phänotyps bei Fibroblasten nachgewiesen, die durch Transformation mit dem v-Src Onkogen induziert worden war (Uehara et al., 1985).

Später wurde eine starke antitumorale Aktivität in vitro (Schulte et an.,1998) und in vivo in Tiermodellen gezeigt (Supko et al., 1995). Immunpräzipitation und Untersuchungen an Affinitätsmatrices zeigten dann, dass der Hauptwirkmechanismus von Geldanamycin eine Bindung an HSP90 involviert (Whitesell et al., 1994; Schulte and Neckers, 1998). Darüber hinaus wurde durch röntgenkristallographische Untersuchungen gezeigt, dass Geldanamycin um die ATP-Bindestelle kompetitiert und die intrinsische ATPase Aktivität von HSP90 hemmt (Prodromou et al., 1997; Panaretou et al., 1998). Dadurch wird die Entstehung des multimeren HSP90 Komplexes, mit seiner Eigenschaft als Chaperon für "client" Proteine zu fungieren, verhindert. Als Konsequenz werden "client" Proteine über den Ubiquitin-Proteasom-Weg abgebaut.

Das Geldanamycin Derivat 17- Allylamino-17-demethoxygeldanamycin (17AAG) zeigte unveränderte Eigenschaft bei der Hemmung von HSP90, der Degradation von "client" Proteinen und antitumoraler Aktivität in Zellkulturen und in Xenograft Tumormodellen (Schulte et al, 1998; Kelland et al, 1999), hatte aber eine deutlich geringere Leberzytotoxizität als Geldanamycin (Page et all 1997).17AAG wird gegenwärtig in PhaseI/II klinischen Studien geprüft.

Radicicol, ein makrozyklisches Antibiotikum, zeigte ebenfalls Revision des v-Src und v-Ha-Ras induzierten malignen Phänotyps von Fibroblasten (Kwon et all 1992; Zhao et al, 1995). Radicicol degradiert eine Vielzahl von Signalproteinen als Konsequenz der HSP90 Hemmung (Schulte et al., 1998). Röntgenkristallographische Untersuchungen zeigten, dass Radicicol ebenfalls an die N-terminale Domäne von HSP90 bindet und die intrinsische ATPase Aktivität hemmt (Roe et al., 1998).

Antibiotika vom Coumarin Typ binden bekannterweise an die ATP Bindestelle des HSP90 Homologs DNA Gyrase in Bakterien. Das Coumarin, Novobiocin, bindet an das Carboxy-terminale Ende von HSP90, also an eine andere Stelle bei HSP90 als die Benzochinon-Ansamycine und Radicicol, welche an das N-terminale Ende von HSP90 binden.(Marcu et al., 2000b).

Die Hemmung von HSP90 durch Novobiocin resultiert in der Degradation einer großen Zahl von HSP90-abhängigen Signalproteinen (Marcu et al., 2000a).

Mit PU3, einem von Purinen abgeleiteten HSP90 Inhibitor konnte die Degradation von Signalproteinen z.B. ERBB2, gezeigt werden. PU3 verursacht Zellzyklus-Arrest und Differenzierung in Brustkrebs-Zelllinien (Chiosis et al., 2001).

### HSP90 als therapeutisches Target

Durch die Beteiligung von HSP90 an der Regulation einer großen Zahl von Signalwegen, die entscheidende Bedeutung am Phänotyp eines Tumors haben, und der Entdeckung, dass gewisse Naturstoffe ihren biologischen Effekt durch Hemmung der Aktivität von HSP90 ausüben, wird HSP90 gegenwärtig als neues Target für die Entwicklung eines Tumortherapeutikum geprüft (Neckers et al., 1999).

Der Hauptmechanismus der Wirkweise von Geldanamycin, 17AAG, und Radicicol beinhaltet die Hemmung der Bindung von ATP an die ATP-Bindestelle am N-terminalen Ende des Proteins und die daraus resultierende Hemmung der intrinsischen ATPase-Aktivität von HSP90 (siehe z.B. Prodromou et al., 1997; Stebbins et al., 1997; Panaretou et al., 1998). Die Hemmung der ATPase-Aktivität von HSP90 verhindert die Rekrutierung von Co-chaperonen und favorisiert die Bildung eines HSP90 Heterokomplexes, der "client" Proteine über den Ubiquitin-Proteasom-Weg der Degradation zuführt (siehe, z.B. Neckers et al., 1999; Kelland et al., 1999). Die Behandlung von Tumorzellen mit HSP90 Inhibitoren führt zur selektiven Degradation wichtiger Proteine mit fundamentaler Bedeutung für Prozesse wie Zellproliferation, Regulation des Zellzyklus und Apoptose. Diese Prozesse sind häufig in Tumoren dereguliert (siehe z.B. Hostein et al., 2001).

Eine attraktive Rationale für die Entwicklung eines Inhibitors von HSP90 ist, dass durch gleichzeitige Degradation mehrerer Proteine, die mit dem transformierten Phänotyp im Zusammenhang stehen, eine starke tumortherapeutische Wirkung erreicht werden kann.

Im einzelnen betrifft die vorliegende Erfindung Verbindungen, die HSP90 hemmen, regulieren und/oder modulieren, Zusammensetzungen, die diese Verbindungen enthalten, sowie Verfahren zu ihrer Verwendung zur Behandlung von HSP90-bedingten Krankheiten, wie Tumorerkrankungen, virale Erkrankungen wie z.B. Hepatitis B (Waxman, 2002); Immunsuppression bei Transplantationen (Bijlmakers, 2000 and Yorgin, 2000); Entzündungsbedingte Erkrankungen (Bucci, 2000) wie Rheumatoide Arthritis, Asthma, Multiple Sklerose, Typ 1 Diabetes, Lupus Erythematodes, Psoriasis und Inflammatory Bowel Disease; Zystische Fibrose (Fuller, 2000); Erkrankungen im Zusammenhang mit Angiogenese (Hur, 2002 and Kurebayashi, 2001) wie z.B. diabetische Retinopathie, Hämangiome, Endometriose und Tumorangiogenese; infektiöse Erkrankungen; Autoimmunerkrankungen; Ischämie; Förderung der Nervenregeneration (Rosen et al., WO 02/09696; Degranco et al., WO 99/51223; Gold, US 6,210,974 B1); fibrogenetische Erkrankungen, wie z.B. Sklerodermie, Polymyositis, systemischer Lupus, Leberzirrhose, Keloidbildung, interstitielle Nephritis und pulmonare Fibrose (Strehlow, WO 02/02123).

Die Erfindung betrifft auch die Verwendung der erfindungsgemäßen Verbindungen zum Schutz normaler Zellen gegen Toxizität, die durch Chemotherapie verursacht ist, sowie die Verwendung bei Krankheiten, wobei Proteinfehlfaltung oder Aggregation ein Hauptkausalfaktor ist, wie z.B. Skrapie, Creutzfeldt-Jakob-Krankheit, Huntington oder Alzheimer (Sittler, Hum. Mol. Genet., 10, 1307, 2001; Tratzelt et al., Proc. Nat. Acad. Sci., 92, 2944, 1995; Winklhofer et al., J. Biol. Chem., 276, 45160, 2001). A. Kamal et al. beschreiben in Trends in Molecular Medicine, Vol. 10 No. 6 June 2004, therapeutische und diagnostische Anwendungen der HSP90 Aktivierung, u.a. zur Behandlung von Krankheiten des Zentralnervensystems und von Herzkreislauferkrankungen.

Die Identifikation von kleinen Verbindungen, die HSP90 spezifisch hemmen, regulieren und/oder modulieren, ist daher wünschenswert und ein Ziel der vorliegenden Erfindung.

Es wurde gefunden, daß die erfindungsgemäßen Verbindungen gemäß Anspruch 1 und ihre Salze bei guter Verträglichkeit sehr wertvolle pharmakologische Eigenschaften besitzen.
Insbesondere zeigen sie inhibierende Eigenschaften des HSP90.

Gegenstand der vorliegenden Erfindung sind deshalb Verbindungen gemäß Anspruch 1 als Arzneimittel und/oder Arzneimittelwirkstoffe bei der Behandlung und/oder Prophylaxe der genannten Erkrankungen und die Verwendung von Verbindungen gemäß Anspruch 1 zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer Verbindungen gemäß Anspruch 1 an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

### STAND DER TECHNIK

In der WO 00/53169 wird die HSP90-Inhibierung mit Coumarin oder einem Coumarinderivat beschrieben.
In der WO 03/041643 A2 sind HSP90-inhibierende Zearalanol-Derivate offenbart.
Andere HSP90-inhibierende Pyrazolderivate, die in 3- oder 5-Stellung durch einen Aromaten substituiert sind, kennt man aus WO 2004/050087 A1 und WO 2004/056782 A1.
In der WO 03/055860 A1 sind 3,4-Diarylpyrazole als HSP90-Inhibitoren beschrieben.
Purinderivate mit HSP90-inhibierenden Eigenschaften sind in der WO 02/36075 A2 offenbart.
In Chemistry of Heterocyclic Compounds 40(2), 183-187 (Februar 2004*)* wird die Verbindung 3-(4-Benzyloxy-4-hydroxy)-5-methyl-4-phenoxy-1H-pyrazol offenbart.
In Database Chemical Abstracts werden zwei 4-(Methoxyphenyl)-1H-pyrazol-Derivate mit den Nummern [716333-47-0] und [716332-28-4] beschrieben.
In der WO 03/055860 und in der WO 2004/050087 werden andere Pyrazolderivate als HSP90 Inhibitoren beschrieben.

Folgende Phenylpyrazole sind unter ihrer CAS Registry Number bekannt:
374909-20-3
3-(2,4-Dihydroxy-phenyl)-4-phenoxy-5-methyl-1*H*-pyrazol,
476609-64-0
3-(2,4-Dihydroxy-phenyl)-4-(4-nitro-phenoxy)-5-methyl-1*H*-pyrazol,
433703-66-3
3-(2,4-Dihydroxy-5-ethyl-phenyl)-4-(4-chlor-phenoxy)-1 H-pyrazol,
433702-50-2
3-(2,4-Dihydroxy-5-ethyl-phenyl)-4-(4-fluor-phenoxy)-5-methyl-1*H*-pyrazol,
433702-41-1
3-(2,4-Dihydroxy-phenyl)-4-(3-phenyl-phenoxy)-5-methyl-1*H*-pyrazol,
433697-01-9
3-(2,4-Dihydroxy-phenyl)-4-(4-phenyl-phenoxy)-1*H*-pyrazol,
433329-33-0
3-(2,4-Dihydroxy-5-ethyl-phenyl)-4-(4-phenyl-phenoxy)-1*H*-pyrazol,
433329-32-9,
3-(2,4-Dihydroxy-5-ethyl-phenyl)-4-(4-isopropyl-phenoxy)-5-methyl-1*H-*pyrazol,
433329-19-2
3-(2-Hydroxy-4-methoxy-phenyl)-4-(4-phenyl-phenoxy)-5-methyl-1*H-*pyrazol,
433328-86-0
3-(2-Hydroxy-4-methoxy-5-ethyl-phenyl)-4-(4-phenyl-phenoxy)-1*H*-pyrazol,
433328-01-9,
3-(2-Hydroxy-4-methoxy-5-ethyl-phenyl)-4-(4-chlor-phenoxy)-5-methyl-1*H-*pyrazol,
431919-95-8
3-(2-Hydroxy-4-methyl-5-ethyl-phenyl)-4-(2-fluor-phenoxy)-5-methyl-1*H-*pyrazol,
431890-54-9
3-(2-Hydroxy-4-methoxy-phenyl)-4-(2-fluor-phenoxy)-1*H-*pyrazol,
431886-77-0
3-(2,4-Dihydroxy-phenyl)-4-(2-fluor-phenoxy)-5-methyl-1*H*-pyrazol,
431072-63-8
3-(2-Hydroxy-4-methoxy-phenyl)-4-(4-fluor-phenoxy)-1*H*-pyrazol,
431056-28-9
3-(2-Hydroxy-4-methoxy-phenyl)-4-(4-chlor-phenoxy)-1*H-*pyrazol,
430470-45-4
3-(2-Hydroxy-4-methoxy-5-ethyl-phenyl)-4-(4-fluor-phenoxy)-1H-pyrazol,
430451-78-8
3-(2,4-Dihydroxy-phenyl)-4-(4-fluor-phenoxy)-5-trifluormethyl-1*H*-pyrazol,
415686-18-9
3-(2,4-Dihydroxy-phenyl)-4-phenoxy-5-trifluormethyl-1*H*-pyrazol,
412962-83-5
3-(2,4-Dihydroxy-phenyl)-4-(4-brom-phenoxy)-1*H*-pyrazol,
394237-90-2
3-(2-Hydroxy-4-methoxy-phenyl)-4-(4-nitro-phenoxy)-5-methyl-1*H*-pyrazol,
394228-95-6
3-(2-Hydroxy-4-methoxy-phenyl)-4-(4-carboxy-phenoxy)-1*H*-pyrazol,
394228-66-1
3-(2-Hydroxy-4-methoxy-6-methyl-phenyl)-4-phenoxy-5-methyl-1*H*-pyrazol,
394228-60-5
3-(2-Hydroxy-4-methoxy-phenyl)-4-(4-n-propyl-phenoxy)-5-methyl-1*H-*pyrazol,
394228-57-0
3-(2-Hydroxy-4-methoxy-phenyl)-4-(4-n-propyl-phenoxy)-5-1*H*-pyrazol,
394228-55-8
3-(2,4-Dihydroxy-phenyl)-4-(4-n-propyl-phenoxy)-1*H*-pyrazol,
394228-50-3
3-(2-Hydroxy-4-methoxy-5-ethyl-phenyl)-4-phenoxy-1*H-*pyrazol,
394228-36-5
3-(2-Hydroxy-4-methoxy-5-ethyl-phenyl)-4-phenoxy-5-methyl-1*H-*pyrazol,
385401-47-8
3-(2-Hydroxy-4-methoxy-phenyl)-4-(2-fluor-phenoxy)-5-methyl-1*H*-pyrazol,
385401-44-5
3-(2-Hydroxy-4-methoxy-phenyl)-4-(4-fluor-phenoxy)-5-methyl-1*H*-pyrazol,
385401-27-4
3-(2,4-Dihydroxy-phenyl)-4-(4-n-propyl-phenoxy)-5-methyl-1*H*-pyrazol,
385401-21-8
3-(2,4-Dihydroxy-6-methyl-phenyl)-4-phenoxy-5-methyl-1 H-pyrazol,
385401-19-4
3-(2,4-Dihydroxy-5-ethyl-phenyl)-4-phenoxy-5-methyl-1*H*-pyrazol,
385401-17-2
3-(2,4-Dihydroxy-5-ethyl-phenyl)-4-phenoxy-1*H*-pyrazol,
383412-00-8
3-(2,4-Dihydroxy-phenyl)-4-(2,4-dichlor-phenoxy)-1*H*-pyrazol,
381230-39-3
3-(2-Hydroxy-4-methoxy-5-ethyl-phenyl)-4-(4-fluor-phenoxy)-5-methyl-1*H-*pyrazol,
374699-23-7
3-(2-Hydroxy-4-methoxy-phenyl)-4-(4-ethoxy-phenoxy)-5-methyl-1*H-*pyrazol,
374699-18-0
3-(2,4-Dihydroxy-phenyl)-4-(4-ethoxy-phenoxy)-5-methyl-1*H*-pyrazol,
374698-43-8
3-(2-Hydroxy-4-methoxy-phenyl)-4-phenoxy-5-methyl-1*H*-pyrazol,
327100-86-7
3-(2-Hydroxy-4-methoxy-phenyl)-4-(4-chlor-phenoxy)-5-methyl-1*H-*pyrazol,
312309-70-9
3-(2-Hydroxy-4-methoxy-phenyl)-4-phenoxy-1*H*-pyrazol,
311809-91-3
3-(2,4-Dihydroxy-phenyl)-4-(2-brom-phenoxy)-5-methyl-1*H*-pyrazol,
310440-73-4
3-(2,4-Dihydroxy-phenyl)-4-(2,4-dichlor-phenoxy)-5-methyl-1*H*-pyrazol,
308298-51-3
3-(2,4-Dihydroxy-phenyl)-4-(4-chlor-phenoxy)-5-methyl-1*H*-pyrazol,
306280-10-4,
3-(2-Hydroxy-4-methoxy-phenyl)-4-(4-methoxycarbonyl-phenoxy)-1*H-*pyrazol,
304880-27-1
3-(2,4-Dihydroxy-phenyl)-4-(4-brom-phenoxy)-5-methyl-1*H-*pyrazol,
300834-71-3
3-(2,4-Dihydroxy-phenyl)-4-(4-ethoxy-phenoxy)-1*H*-pyrazol,
297147-95-6
3-(2,4-Dihydroxy-phenyl)-4-(4-fluor-phenoxy)-5-methyl-1*H*-pyrazol,
294668-76-1
3-(2,4-Dihydroxy-phenyl)-4-(4-fluor-phenoxy)-1*H*-pyrazol,
263744-90-7
3-(2,4-Dihydroxy-phenyl)-4-phenoxy)-1*H*-pyrazol,
510766-37-7
3-(2,4-Dihydroxy-phenyl)-4-(4-methoxycarbonyl-phenoxy)-1*H*-pyrazol.

In der WO 01/72779 sind Purinverbindungen beschrieben, sowie deren Verwendung zur Behandlung von GRP94 (Homolog oder Paralog zu HSP90)-bedingten Krankheiten, wie Tumorerkrankungen, wobei das Krebsgewebe ein Sarkom oder Karzinom umfasst, ausgewählt aus der Gruppe, bestehend aus Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung.

In der WO 01/72779 ist weiterhin die Verwendung der dort genannten Verbindungen zur Behandlung von viralen Erkrankungen offenbart, wobei das virale Pathogen ausgewählt ist aus der Gruppe, bestehend aus Hepatitis Typ A, Hepatitis Typ B, Hepatitis Typ C, Influenza, Varicella, Adenovirus, Herpes-Simplex Typ I (HSV-I), Herpes Simplex Typ II (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masernvirus, Rötelnvirus, Poliovirus, menschliches Immunschwächevirus Typ I (HIV-I) und menschliches Immunschwächevirus Typ II (HIV-II).
In der WO 01/72779 ist ferner die Verwendung der dort genannten Verbindungen zur GRP94-Modulation beschrieben, wobei die modulierte biologische GRP94-Aktivität eine Immunreaktion in einem Individuum, Proteintransport vom endoplasmatischen Retikulum, Genesung vom hypoxischen/anoxischen Stress, Genesung von Unterernährung, Genesung von Hitzestress, oder Kombinationen davon, hervorruft, und/oder wobei die Störung eine Art Krebs ist, eine Infektionserkrankung, eine Störung, die mit einem gestörten Proteintransport vom endoplasmatischen Retikulum, einer Störung, die mit Ischämie / Reperfusion einhergeht, oder Kombinationen davon, wobei die die mit Ischämie / Reperfusion einhergehende Störung eine Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

In der WO 01/72779 ist schließlich die Verwendung einer wirksamen Menge eines GRP94-Proteinmodulators zur Herstellung eines Medikamentes bechrieben, zum Verändern einer anschließenden zellulären Reaktion auf einen ischämischen Zustand bei einer Gewebestelle in einem Individuum, durch Behandlung der Zellen an der Gewebestelle mit dem GRP94-Proteinmodulator, damit die GRP94-Aktivität in Zellen dermaßen verstärkt wird, dass eine anschließende zelluläre Reaktion auf einen ischämischen Zustand verändert wird, wobei die anschließende ischämische Bedingung vorzugsweise die Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer Krankheit, Chorea Huntington, amyotropher lateraler Sklerose (ALS) oder Stress beim Neugeborenen ist, oder wobei die Gewebestelle das Donatorgewebe für eine Transplantation ist.

### Weitere Literatur:

Argon Y and Simen BB. 1999 "Grp94, an ER chaperone with protein and peptide binding properties", Semin. Cell Dev. Biol., Vol. 10, pp. 495-505.
Bijlmakers M-JJE, Marsh M. 2000 "Hsp90 is essential for the synthesis and subsequent membrane association, but not the maintenance, of the Srckinase p56lck", Mol. Biol. Cell, Vol. 11 (5), pp. 1585-1595.
Bucci M; Roviezzo F; Cicala C; Sessa WC, Cirino G. 2000 "Geldanamycin, an inhibitor of heat shock protein 90 (Hsp90) mediated signal transduction has anti-inflammatory effects and interacts with glucocorticoid receptor in vivo", Brit. J. Pharmacol., Vol 131(1), pp. 13-16.
Carreras CW, Schirmer A, Zhong Z, Santi VS. 2003 "Filter binding assay for the geldanamycin-heat shock protein 90 interaction", Analytical Biochem., Vot 317, pp 40-46.
Chen C-F, Chen Y, Dai KD, Chen P-L, Riley DJ and Lee W-H. 1996 "A new member of the hsp90 family of molecular chaperones interacts with the retinoblastoma protein during mitosis and after heat shock", Mol. Cell. Biol., Vol. 16, pp. 4691-4699.
Chiosis G, Timaul MN, Lucas B, Munster PN, Zheng FF, Sepp-Lozenzino L and Rosen N. 2001 "A small molecule designed to bind to the adenine nucleotide pocket of HSP90 causes Her2 degradation and the growth arrest and differentiation of breast cancer cells", Chem. Biol., Vol. 8, pp. 289-299.
Chiosis G, Lucas B, Shtil A, Huezo H, Rosen N 2002 "Development of a purine-scaffold novel class of HSP90 binders that inhibit the proliferation of cancer cells and induce the degradation of her2 tyrosine kinase". Bioorganic Med. Chem., Vol 10, pp 3555-3564.
Conroy SE and Latchman DS. 1996 "Do heat shock proteins have a role in breast cancer?", Brit. J. Cancer, Vol. 74, pp. 717-721.
Felts SJ, Owen BAL, Nguyen P, Trepel J, Donner DB and Toft DO. 2000 "The HSP90-related protein TRAP1 is a mitochondrial protein with distinct functional properties", J. Biol. Chem., Vol. 5, pp. 3305-331 2.
Fuller W, Cuthbert AW. 2000 "Post-translational disruption of the delta F508 cystic fibrosis transmembrane conductance regulator (CFTR)-molecular Chaperone complex with geldanamycin stabilizes delta F508 CFTR in the rabbit reticulocyte lysate", J. Biol. Chem., Vol. 275(48), pp. 37462-37468.
Hickey E, Brandon SE, Smale G, Lloyd D and Weber LA. 1999 "Sequence and regulation of a gene encoding a human 89-kilodalton heat shock protein", Mol. Cell. Biol., Vol. 9, pp. 2615-2626.
Hoang AT, Huang J, Rudra-Gonguly N, Zheng J, Powell WC, Rabindron SK, Wu C and Roy-Burman P. 2000 "A novel association between the human heat shock transcription factor 1 (HSF1) and prostate adenocarcinoma, Am. J. Pathol., Vol. 156, pp. 857-864.
Hostein I, Robertson D, Di Stefano F, Workman P and Clarke PA. 2001 "Inhibition of signal transduction by the HSP90 inhibitor 17-allylamino-1 7-demethoxygeldanamycin results in cytostasis and apoptosis", Cancer Res., Vol. 61, pp. 4003-4009.
Hur E, Kim H-H, Choi SM, Kim JH, Yim S, Kwon HJ, Choi Y, Kim DK, Lee M-0, Park H. 2002 "Reduction of hypoxia-induced transcription through the repression of hypoxia-inducible factor-1α/aryl hydrocarbon receptor nuclear translocator DNA binding by the 90-kDa heat-shock protein inhibitor radicicol", Mol. Pharmacol., Vol 62(5), pp. 975-982.
Jameel A, Skilton RA, Campbell TA, Chander SK, Coombes RC and Luqmani YA. 1992 "Clinical
Jolly C and Morimoto RI. 2000 "Role of the heat shock response and molecular chaperones in oncogenesis and cell death", J. Natl. Cancer Inst., Vol. 92, pp. 1564-1572.
Kawanishi K, Shiozaki H, Doki Y, Sakita I, Inoue M, Yano M, Tsujinata T, Shamma A and Monden M. 1999 "Prognostic significance of heat shock proteins 27 and 70 in patients with squamous cell carcinoma of the esophagus", Cancer, Vol. 85, pp. 1649-1657.
Kelland LR, Abel G, McKeage MJ, Jones M, Goddard PM, Valenti M, Murrer BA, and Harrap KR. 1993 "Preclinical antitumour evaluation of bisacetalo-amino-dichloro-cyclohexylamine platinum (IV): an orally active platinum drug", Cancer Research, Vol. 53, pp. 2581 - 2586.
Kelland LR, Sharp SY, Rogers PM, Myers TG and Workman P. 1999 "DT-diaphorase expression and tumor cell sensitivity to 17-allylamino,17-demethoxygeldanamycin, an inhibitor of heat shock protein 90", J. Natl. Cancer Inst., Vol. 91, pp. 1940-1949.
Kurebayashi J, Otsuki T, Kurosumi M, Soga S, Akinaga S, Sonoo, H. 2001 "A radicicol derivative, KF58333, inhibits expression of hypoxia-inducible factor-1α and vascular endothelial growth factor, angiogenesis and growth of human breast cancer xenografts", Jap. J. Cancer Res.,Vol. 92( 12), 1342-1351.
Kwon HJ, Yoshida M, Abe K, Horinouchi S and Bepple T. 1992 "Radicicol, an agent inducing the reversal of transformed phentoype of srctransformed fibroblasts, Biosci., Biotechnol., Biochem., Vol. 56, pp. 538-539.
Lebeau J, Le Cholony C, Prosperi MT and Goubin G. 1991 "Constitutive overexpression of 89 kDa heat shock protein gene in the HBL100 mammary cell line converted to a tumorigenic phenotype by the EJE24 Harvey-ras oncogene", Oncogene, Vol. 6, pp. 1125-1132.
Marcu MG, Chadli A, Bouhouche I, Catelli M and Neckers L. 2000a "The heat shock protein 90 antagonist novobiocin interacts with a previously unrecognized ATP-binding domain in the carboxyl terminus of the chaperone", J. Biol. Chem., Vol. 275, pp. 37181-37186.
Marcu MG, Schulte TW and Neckers L. 2000b "Novobiocin and related coumarins and depletion of heat shock protein 90-dependent signaling proteins", J. Natl. Cancer Inst., Vol. 92, pp. 242-248.
Martin KJ, Kritzman BM, Price LM, Koh B, Kwan CP, Zhang X, MacKay A, O'Hare MJ, Kaelin CM, Mutter GL, Pardee AB and Sager R. 2000 "Linking gene expression patterns to therapeutic groups in breast cancer", Cancer Res., Vol. 60, pp. 2232-2238.
Neckers L, Schulte TW and Momnaaugh E. 1999 "Geldanamycin as a potential anti-cancer agent: its molecular target and biochemical activity", Invest. New Druqs, Vol. 17, pp. 361-373.
Page J, Heath J, Fulton R, Yalkowsky E, Tabibi E, Tomaszewski J, Smith A and Rodman L. 1997 "Comparison of geldanamycin (NSC-122750) and 17-allylaminogeldanamycin (NSC-330507D) toxicity in rats", Proc. Am. Assoc. Cancer Res., Vol. 38, pp. 308.
Panaretou B, Prodromou C, Roe SM, OBrien R, Ladbury JE, Piper PW and Pearl LH. 1998 "ATP binding and hydrolysis are essential to the function of the HSP90 molecular chaperone in vivo", EMBO J., Vol. 17, pp. 4829-4836.
Pratt WB. 1997 "The role of the HSP90-based chaperone system in signal transduction by nuclear receptors and receptors signalling via MAP kinase", Annu. Rev. Pharmacol. Toxicol., Vol. 37, pp. 297-326.
Prodromou C, Roe SM, O'Brien R, Ladbury JE, Piper PW and Pearl LH. 1997 "Identification and structural characterization of the ATP/ADP-binding site in the HSP90 molecular chaperone", Cell, Vol. 90, pp. 65-75.
Prodromou C, Panaretou B, Chohan S, Siligardi G, O'Brien R, Ladbury JE, Roe SM, Piper PW and Pearl LH. 2000 "The ATPase cycle of HSP90 drives a molecular "clamp" via transient dimerization of the N-terminal domains", EMBO J., Vol. 19, pp. 4383-4392.
Roe SM, Prodromou C, O'Brien R, Ladbury JE, Piper PW and Pearl LH. 1999 "Structural basis for inhibition of the HSP90 molecular chaperone by the antitumour antibiotics radicicol and geldanamycin", J. Med. Chem., Vol. 42, pp. 260-266.
Rutherford SL and Lindquist S. 1998 "HSP90 as a capacitor for morphological evolution. Nature, Vol. 396, pp. 336-342.
Schulte TW, Akinaga S, Murakata T, Agatsuma T, Sugimoto S, Nakano H, Lee YS, Simen BB, Argon Y, Felts S, Toft DO, Neckers LM and Sharma SV. 1999 "Interaction of radicicol with members of the heat shock protein 90 family of molecular chaperones", Mol. Endocrinoloqy, Vol. 13, pp. 1435-1448.
Schulte TW, Akinaga S, Soga S, Sullivan W, Sensgard B, Toft D and Neckers LM. 1998 "Antibiotic radicicol binds to the N-terminal domain of HSP90 and shares important biologic activities with geldanamcyin", Cell Stress and Chaperones, Vol. 3, pp. 100-108.
Schulte TW and Neckers LM. 1998 "The benzoquinone ansamycin 17-allylamino-17-demethoxygeldanamcyin binds to HSP90 and shares important biologic activities with geldanamycin", Cancer Chemother. Pharmacol., Vol. 42, pp. 273-279.
Smith DF. 2001 "Chaperones in signal transduction", in: Molecular chaperones in the cell (P Lund, ed.; Oxford University Press, Oxford and NY), pp. 165-178.
Smith DF, Whitesell L and Katsanis E. 1998 "Molecular chaperones: Biology and prospects for pharmacological intervention", Pharmacological Reviews, Vol. 50, pp. 493-513.
Song HY, Dunbar JD, Zhang YX, Guo D and Donner DB. 1995 "Identification of a protein with homology to hsp90 that binds the type 1 tumour necrosis factor receptor", J. Biol. Chem., Vol. 270, pp. 3574-3581.
Stebbins CE, Russo A, Schneider C, Rosen N, Hartl FU and Pavletich NP. 1997 "Crystal structure of an HSP90-geldanamcyin complex: targeting of a protein chaperone by an antitumor agent", Cell, Vol. 89, pp. 239-250.
Supko JG, Hickman RL, Grever MR and Malspeis L. 1995 "Preclinical pharmacologic evaluation of geldanamycin as an antitumour agent", Cancer Chemother. Pharmacol., Vol. 36, pp. 305-315.
Tytell M and Hooper PL. 2001 "Heat shock proteins: new keys to the development of cytoprotective therapies", Emerging Therapeutic Tarqets, Vol. 5, pp. 267-287.
Uehara U, Hori M, Takeuchi T and Umezawa H. 1986 "Phenotypic change from transformed to normal induced by benzoquinoid ansamycins accompanies inactivation of p6Osrc in rat kidney cells infected with Rous sarcoma virus", Mol. Cell. Biol., Vol. 6, pp. 21 98-2206.
Waxman, Lloyd H. Inhibiting hepatitis C virus processing and replication. (Merck & Co., Inc., USA). PCT Int. Appl. (2002), WO 0207761
Whitesell L, Mimnaugh EG, De Costa B, Myers CE and Neckers LM. 1994 "Inhibition of heat shock protein HSP90-pp60v-src heteroprotein complex formation by benzoquinone ansamycins: essential role for stress proteins in oncogenic transformation", Proc. Natl. Acad. Sci. USA., Vol. 91, pp. 8324-8328.
Yorgin et al. 2000 "Effects of geldanamycin, a heat-shock protein 90-binding agent, on T cell function and T cell nonreceptor protein tyrosine kinases", J. Immunol., Vol 164(6), pp. 2915-2923.
Young JC, Moarefi I and Hartl FU. 2001 "HSP90: a specialized but essential protein-folding tool", J. Cell. Biol., Vol. 154, pp. 267-273.
Zhao JF, Nakano H and Sharma S. 1995 "Suppression of RAS and MOS transformation by radicicol", Oncoqene, Vol. 11, pp. 161 -173.

### ZUSAMMENFASSUNG DER ERFINDUNG

Die Erfindung betrifft einzelne Verbindungen gemäß Anspruch 1 umfaßt von der Formel I 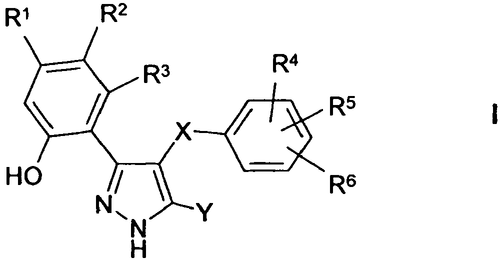 worin
- R¹: OH, OCH₃, OCF₃, OCHF₂, OBzl, OAc, p-Methoxybenzyloxy,SH, S(O)ₘCH₃, SO₂NH₂, Hal, CF₃ oder CH₃,
- R², R³: jeweils unabhängig voneinander H, Hal, CN, NO₂, A, Alk, (CH₂)ₙAr, (CH₂)ₙHet, COOH, COOA, COOAr, COOHet, CONH₂, CONHA, CONAA', CONHAr, CONAAr, CON(Ar)₂, CONHHet, CON(Het)₂, NH₂, NHA, NHAr, NHHet, NAA', NHCOA, NACOA', NHCOAr, NHCOHet, NHCOOA, NHCOOAr, NHCOOHet, NHCONHA, NHCONHAr, NHCONHHet, OH, OA, OAr, OHet, SH, S(O)ₘA, S(O)ₘAr. S(O)ₘHet, SO₂NH₂, SO₂NHA, SO₂NAA', SO₂NH(CH₂)ₙAr, SO₂NAAr, SO₂NH(CH₂)ₙHet, SO₂N(Ar)₂ oder SO₂N(Het)₂,
- R⁴, R⁵, R⁶: jeweils unabhängig voneinander H, Hal, CN, NO₂, A, Alk, (CH₂)ₙAr, (CH₂)ₙHet, COOH, COOA, COOAr, COOHet, CONH₂, CONHA, CONAA', CONHAr, CONAAr, CON(Ar)₂, CONHHet, CON(Het)₂, NH₂, NHA, NHAr, NHHet, NAA', NHCOA, NHCONH₂, NACOA', NHCOAr, NHCOHet, NHCOOA, NHCOOAr, NHCOOHet, NHCONHA, NHCONHAr, NHCONHHet, OH, OA, OAr, OHet, SH, S(O)ₘA, S(O)ₘAr, S(O)ₘHet, SO₂NH₂, SO₂NHA, SO₂NAA', SO₂NHAr, SO₂NAAr, SO₂NHHet, SO₂N(Ar)₂ oder SO₂N(Het)₂,
- R⁴ und R⁵: zusammen auch OCH₂O, OCH₂CH₂O, NH-CH=CH oder CH=CH-NH,
- X: O, S, SO oder SO₂,
- Y: H, A, Ar oder Het,
- A, A': jeweils unabhängig voneinander unverzweigtes oder verzweigtes Alkyl mit 1-10 C-Atomen, worin eine, zwei oder drei CH₂-Gruppen durch O, S, SO, SO₂, NH, NR⁸ und/oder durch -CH=CH-Gruppen und/oder auch 1-5 H-Atome durch F, Cl und/oder R⁷ ersetzt sein können, Alk oder cyclisches Alkyl mit 3-7 C-Atomen,
- A und A': zusammen auch eine Alkylenkette mit 2, 3, 4, 5 oder 6 C-Atomen, worin eine CH₂-Gruppe durch O, S, SO, SO₂, NH, NR⁸, NCOR⁸ oder NCOOR⁸ ersetzt sein kann,
- Alk: Alkenyl mit 2-6 C-Atomen,
- R⁷: COOR⁹, CONR⁹R¹⁰, NR⁹R¹⁰, NHCOR⁹, NHCOOR⁹ oder OR⁹,
- R⁸: Cycloalkyl mit 3-7 C-Atomen, Cycloalkylalkylen mit 4-10 C-Atomen, Alk oder unverzweigtes oder verzweigtes Alkyl mit 1-6 C-Atomen, worin eine, zwei oder drei CH₂-Gruppen durch O, S, SO, SO₂, NH und/oder auch 1-5 H-Atome durch F und/oder Cl ersetzt sein können,
- R⁹, R¹⁰: jeweils unabhängig voneinander H oder Alkyl mit 1-5 C-Atomen, worin 1-3 CH₂-Gruppen durch O, S, SO, SO₂, NH, NMe oder NEt und/oder auch 1-5 H-Atome durch F und/oder Cl ersetzt sein können,
- R⁹ und R¹⁰: zusammen auch eine Alkylenkette mit 2, 3, 4, 5 oder 6 C-Atomen, worin eine CH₂-Gruppe durch O, S, SO, SO₂, NH; NR⁸, NCOR⁸ oder NCOOR⁸ ersetzt sein kann,
- Ar: unsubstituiertes oder ein-, zwei- oder dreifach durch Hal, A, OR¹¹, N(R¹¹)₂, NO₂, CN, Phenyl, CON(R¹¹)₂, NR¹¹COA, NR¹¹CON(R¹¹)₂, NR¹¹SO₂A, COR¹¹, SO₂N(R¹¹)₂, S(O)ₘA,-[C(R¹¹)₂]ₙ-COOR¹¹ und/oder -O[C(R¹¹)₂]ₒ-COOR¹¹ substituiertes Phenyl, Naphthyl oder Biphenyl,
- Het: einen ein- oder zweikernigen gesättigten, ungesättigten oder aromatischen Heterocyclus mit 1 bis 4 N-, O- und/oder S-Atomen, der ein-, zwei- oder dreifach durch Hal, A, (CH₂)ₙOR¹¹, (CH₂)ₙN(R¹¹)₂, NO₂, CN, COOR¹¹, CON(R¹¹)₂, NR¹¹COA, NR¹¹SO₂A, COR¹¹, SO₂NR¹¹, S(O)ₘA, =S, =NR¹¹ und/oder =O (Carbonylsauerstoff) substituiert sein kann,
- R¹¹: H oder A,
- Hal: F, Cl, Br oder I,
- m: 0, 1 oder 2,
- n: 0, 1, 2, 3 oder 4,
- o: 1, 2 oder 3
bedeuten,
sowie ihre pharmazeutisch verwendbaren Salze, Solvate, Tautomere und Stereoisomere, einschließlich deren Mischungen in allen Verhältnissen, wobei Verbindungen der Formel I, worin
- R¹: OH oder OCH₃,
- R²: H oder Ethyl,
- R³: H oder Methyl,
- R⁴: H, NO₂, F, Cl, Br, Phenyl, i-Propyl, n-Propyl, COOH, COOCH₃ oder Ethoxy,
- R⁵: H oder Cl,
- R⁶: H,
- X: O,
- Y: H, CH₃ oder CF₃
bedeuten,
ausgenommen sind.

Der Disclaimer umfaßt die bekannten Phenylpyrazole.

Gegenstand der Erfindung sind die Verbindungen der Formel I gemäß Anspruch 1 und ihre Salze sowie ein Verfahren zur Herstellung von Verbindungen der Formel I gemäß Anspruch 1 sowie ihrer pharmazeutisch verwendbaren Solvate, Salze, Tautomere und Stereoisomere, dadurch gekennzeichnet, daß man
a) eine Verbindung der Formel II 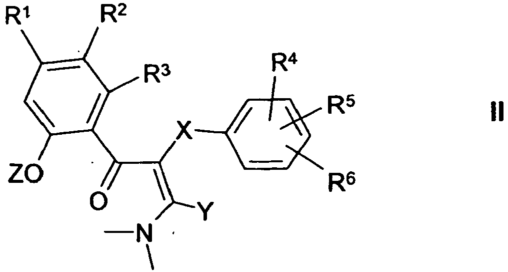
worin
R¹, R², R³, R⁴, R⁵, R⁶, X und Y die in Anspruch 1 entsprechenden Bedeutungen haben,
und Z H oder Methyl bedeutet,
mit
einer Verbindung der Formel III

H₂N-NH₃⁺OH⁻ III

umsetzt,
anschließend gegebenenfalls durch Etherspaltung die erhaltene Verbindung, worin Z Methyl bedeutet, in eine Verbindung der Formel I umwandelt, worin Z H bedeutet,
und/oder daß man in einer Verbindung der Formel I einen oder mehrere Rest(e) R¹,R², R³, R⁴, R⁵ und/oder R⁶ in einen oder mehrere Rest(e) R¹,R², R³, R⁴, R⁵ und/oder R⁶ umwandelt,
indem man beispielsweise
i) eine Nitrogruppe zu einer Aminogruppe reduziert,
ii) eine Estergruppe zu einer Carboxygruppe hydrolysiert,
iii) eine Aminogruppe durch reduktive Aminierung in ein alkyliertes Amin umwandelt,
iv) ein Säurechlorid in ein Amid überführt,
und/oder
eine Base oder Säure der Formel I in eines ihrer Salze umwandelt.

Gegenstand der Erfindung sind auch die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Die erfindungsgemäßen Verbindungen der Formel I können auch in der tautomeren Form der Formel Ia vorliegen

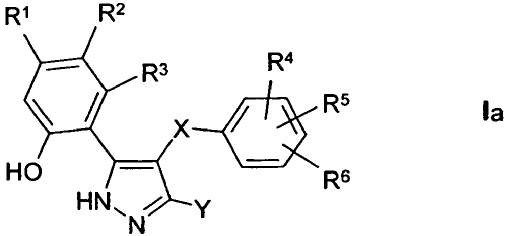

Unter pharmazeutisch verwendbaren Derivaten versteht man z.B. die Salze der erfindungsgemäßen Verbindungen als auch sogenannte Prodrug-Verbindungen.
Unter Prodrug-Derivaten versteht man mit z. B. Alkyl- oder Acylgruppen, Zuckern oder Oligopeptiden abgewandelte Verbindungen der Formel I, die im Organismus rasch zu den wirksamen erfindungsgemäßen Verbindungen gespalten werden.

Hierzu gehören auch bioabbaubare Polymerderivate der erfindungsgemäßen Verbindungen, wie dies z. B. in Int. J. Pharm. 115, 61-67 (1995) beschrieben ist.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.
Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.

Die Bezeichnung "therapeutisch wirksame Menge" umfaßt auch die Mengen, die wirkungsvoll sind, die normale physiologische Funktion zu erhöhen.

Gegenstand der Erfindung sind auch Mischungen der erfindungsgemäßen Verbindungen der Formel I gemäß Anspruch 1, z.B. Gemische zweier Diastereomerer z.B. im Verhältnis 1:1, 1:2, 1:3, 1:4, 1:5, 1:10, 1:100 oder 1:1000.
Besonders bevorzugt handelt es sich dabei um Mischungen stereoisomerer Verbindungen.

Für alle Reste, die mehrfach auftreten, gilt, daß deren Bedeutungen unabhängig voneinander sind.

Vor- und nachstehend haben die Reste bzw. Parameter R¹, R², R³, R⁴, R⁵ und R⁶ die bei der Formel I angegebenen Bedeutungen, falls nicht ausdrücklich etwas anderes angegeben ist.

A bzw. A' bedeutet vorzugsweise Alkyl, ist unverzweigt (linear) oder verzweigt, und hat 1, 2, 3, 4, 5, 6, 7, 8, 9 oder 10 C-Atome. A bzw. A' bedeutet bedeutet besonders bevorzugt Methyl, Ethyl, Propyl, Isopropyl, Butyl, Isobutyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1- , 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl.
A bzw. A' bedeutet ganz besonders bevorzugt Alkyl mit 1, 2, 3, 4, 5 oder 6 C-Atomen, vorzugsweise Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.
A bzw. A' bedeutet auch Cycloalkyl. Cycloalkyl bedeutet vorzugsweise Cyclopropyl, Cyclobutyl, Cylopentyl, Cyclohexyl oder Cycloheptyl.
A bzw. A' bedeutet auch Alk. Alk bedeutet Alkenyl mit 2-6 C-Atomen, wie z.B. Vinyl oder Propenyl.

Ac bedeutet Acetyl, Bzl bedeutet Benzyl, Ms bedeutet -SO₂CH₃.

R¹ bedeutet vorzugsweise OH, OCH₃ oder SH, besonders bevorzugt OH oder OCH₃, ferner auch OCF₃, OCHF₂.
R², R³ bedeuten vorzugsweise jeweils unabhängig voneinander H, Hal, A, OA, OH, SO₂NH(CH₂)ₙAr oder SO₂NH(CH₂)ₙHet.
R² bedeutet besonders bevorzugt H, A, Hal oder SO₂NH(CH₂)ₙAr oder SO₂NH(CH₂)ₙHet.
R³ bedeutet besonders bevorzugt OH oder OA.
R⁴, R⁵, R⁶ bedeuten vorzugsweise jeweils unabhängig voneinander H, Hal, CN, A, COOH, COOA, CONH₂, NHCOA, NHCONH₂, OH, OA, NAA', NH₂, SO₂NH₂, SO₂NHA oder SO₂NAA';
R⁴ und R⁵ bedeuten zusammen auch OCH₂O, OCH₂CH₂O, NH-CH=CH oder CH=CH-NH.
R⁷ bedeutet vorzugsweise COOR⁹, wie z.B. COOH oder COOCH₃; CONR⁹R¹⁰, wie z.B. CONH₂; NR⁹R¹⁰, wie z.B. Amino, Methylamino oder Dimethylamino; NHCOR⁹, NHCOOR⁹ oder OR⁹, wie z.B. Hydroxy oder Methoxy.
R⁹, R¹⁰ bedeuten vorzugsweise jeweils unabhängig voneinander H oder Alkyl mit 1-5 C-Atomen, worin 1-5 H-Atome durch F und/oder Cl ersetzt sein können.

Ar bedeutet z.B. Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Nitrophenyl; o-, m-oder p-Aminophenyl, o-, m- oder p-(N-Methylamino)-phenyl, o-, m- oder p-(N-Methylaminocarbonyl)-phenyl, o-, m- oder p-Acetamidophenyl, o-, m-oder p-Methoxyphenyl, o-, m- oder p-Ethoxyphenyl, o-, m- oder p-Ethoxycarbonylphenyl, o-, m- oder p-(N,N-Dimethylamino)-phenyl, o-, m- oder p-(N,N-Dimethylaminocarbonyl)-phenyl, o-, m- oder p-(N-Ethylamino)-phenyl, o-, m- oder p-(N,N-Diethylamino)-phenyl, o-, m- oder p-Fluorphenyl, o-, m-oder p-Bromphenyl, o-, m- oder p- Chlorphenyl, o-, m- oder p-(Methylsulfonamido)-phenyl, o-, m- oder p-(Methylsulfonyl)-phenyl, o-, m- oder p-Cyanphenyl, o-, m- oder p-Ureidophenyl, o-, m- oder p-Formylphenyl, o-, m- oder p-Acetylphenyl, o-, m- oder p-Aminosulfonylphenyl, o-, m- oder p-Carboxyphenyl, o-, m- oder p-Carboxymethyl-phenyl, o-, m- oder p-Carboxymethoxy-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2,4- oder 2,5-Dinitrophenyl, 2,5- oder 3,4-Dimethoxyphenyl, 3-Nitro-4-chlorphenyl, 3-Amino-4-chlor-, 2-Amino-3-chlor-, 2-Amino-4-chlor-, 2-Amino-5-chlor- oder 2-Amino-6-chlorphenyl, 2-Nitro-4-N,N-dimethylamino- oder 3-Nitro-4-N,N-dimethylamino-phenyl, 2,3-Diaminophenyl, 2,3,4-, 2,3,5-, 2,3,6-, 2,4,6- oder 3,4,5-Trichlorphenyl, 2,4,6-Trimethoxyphenyl, 2-Hydroxy-3,5-dichlorphenyl, p-lodphenyl, 3,6-Dichlor-4-aminophenyl, 4-Fluor-3-chlorphenyl, 2-Fluor-4-bromphenyl, 2,5-Difluor-4-bromphenyl, 3-Brom-6-methoxyphenyl, 3-Chlor-6-methoxyphenyl, 3-Chlor-4-acetamidophenyl, 3-Fluor-4-methoxyphenyl, 3-Amino-6-methylphenyl, 3-Chlor-4-acetamidophenyl oder 2,5-Dimethyl-4-chlorphenyl.

Ar bedeutet vorzugsweise z.B. unsubstituiertes oder ein-, zwei- oder dreifach durch Hal, A, OR¹¹, SO₂A, COOR¹¹ oder CN substituiertes Phenyl, ganz besonders bevorzugt unsubstituiertes oder ein-, zwei- oder dreifach durch Hal und/oder A substituiertes Phenyl.

Het bedeutet, ungeachtetet weiterer Substitutionen, z.B. 2- oder 3-Furyl, 2- oder 3-Thienyl, 1-, 2- oder 3-Pyrrolyl, 1-, 2, 4- oder 5-Imidazolyl, 1-, 3-, 4-oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1- oder 5-Tetrazolyl, 1,2,3-Oxadiazol-4- oder -5-yl, 1,2,4-Oxadiazol-3- oder -5-yl, 1,3,4-Thiadiazol-2- oder -5-yl, 1,2,4-Thiadiazol-3- oder -5-yl, 1,2,3-Thiadiazol-4- oder -5-yl, 3- oder 4-Pyridazinyl, Pyrazinyl, 1-, 2-, 3-, 4-, 5-, 6- oder 7-Indolyl, 4- oder 5-Isoindolyl, 1-, 2-, 4- oder 5-Benzimidazolyl, 1-, 2-, 3-, 4-, 5-, 6- oder 7-Indazolyl, 1-, 3-, 4-, 5-, 6- oder 7-Benzopyrazolyl, 2-, 4-, 5-, 6- oder 7-Benzoxazolyl, 3-, 4-, 5-, 6- oder 7- Benzisoxazolyl, 2-, 4-, 5-, 6- oder 7-Benzothiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl, 5- oder 6-Chinoxalinyl, 2-, 3-, 5-, 6-, 7- oder 8-2H-Benzo-[1,4]oxazinyl, weiter bevorzugt 1,3-Benzodioxol-5-yl, 1,4-Benzodioxan-6-yl, 2,1,3-Benzothiadiazol-4- oder -5-yl oder 2,1,3-Benzoxadiazol-5-yl.
Die heterocyclischen Reste können auch teilweise oder vollständig hydriert sein.
Het kann also z. B. auch bedeuten 2,3-Dihydro-2-, -3-, -4- oder -5-furyl, 2,5-Dihydro-2-, -3-, -4- oder 5-furyl, Tetrahydro-2- oder -3-furyl, 1,3-Dioxolan-4-yl, Tetrahydro-2- oder -3-thienyl, 2,3-Dihydro-1-, -2-, -3-, -4- oder -5-pyrrolyl, 2,5-Dihydro-1-, -2-, -3-, -4- oder -5-pyrrolyl, 1-, 2- oder 3-Pyrrolidinyl, Tetrahydro-1-, -2- oder -4-imidazolyl, 2,3-Dihydro-1-, -2-, -3-, -4- oder -5-pyrazolyl, Tetrahydro-1-, -3- oder -4-pyrazolyl, 1,4-Dihydro-1-, -2-, -3- oder -4-pyridyl, 1,2,3,4-Tetrahydro-1-, -2-, -3-, -4-, -5- oder -6-pyridyl, 1-, 2-, 3- oder 4-Piperidinyl, 2-, 3- oder 4-Morpholinyl, Tetrahydro-2-, -3- oder-4-pyranyl, 1,4-Dioxanyl, 1,3-Dioxan-2-, -4- oder -5-yl, Hexahydro-1-, -3-oder -4-pyridazinyl, Hexahydro-1-, -2-, -4- oder -5-pyrimidinyl, 1-, 2- oder 3-Piperazinyl, 1,2,3,4-Tetrahydro-1-, -2-, -3-, -4-, -5-, -6-, -7- oder-8-chinolyl, 1,2,3,4-Tetrahydro-1-,-2-,-3-, -4-, -5-, -6-, -7- oder -8-isochinolyl, 2-, 3-, 5-, 6-, 7- oder 8- 3,4-Dihydro-2H-benzo[1,4]oxazinyl, weiter bevorzugt 2,3-Methylendioxyphenyl, 3,4-Methylendioxyphenyl, 2,3-Ethylendioxyphenyl, 3,4-Ethylendioxyphenyl, 3,4-(Difluormethylendioxy)phenyl, 2,3-Dihydrobenzofuran-5- oder 6-yl, 2,3-(2-Oxo-methylendioxy)-phenyl oder auch 3,4-Dihydro-2H-1,5-benzodioxepin-6- oder -7-yl, ferner bevorzugt 2,3-Dihydro-benzofuranyl oder 2,3-Dihydro-2-oxo-furanyl.

Het bedeutet vorzugsweise einen einkernigen gesättigten, ungesättigten oder aromatischen Heterocyclus mit 1 bis 2 N- und/oder O-Atomen, der unsubstituiert oder ein-, zwei- oder dreifach durch A, Hal, OH und/oder OA substituiert sein kann.
Het bedeutet besonders bevorzugt einen einkernigen gesättigten Heterocyclus mit 1 bis 2 N- und/oder O-Atomen, der unsubstituiert oder ein- oder zweifach durch A substituiert sein kann.
In einer weiteren Ausführungsform bedeutet Het ganz besonders bevorzugt Pyrrolidinyl, Piperidinyl, Morpholinyl oder Piperazinyl.

In einer weiteren Ausführungsform bedeutet Het besonders bevorzugt unsubstituiertes oder ein-, zwei- oder dreifach durch A, Hal, OH und/oder OA substituiertes Furyl, Thienyl, Pyrrolyl, Imidazolyl, Pyridyl, Pyrimidinyl, Pyrazolyl, Thiazolyl, Indolyl, Pyrrolidinyl, Piperidinyl, Morpholinyl oder Piperazinyl.

In einen weiteren Ausführungsform bedeutet Het vorzugsweise einen einkernigen gesättigten, ungesättigten oder aromatischen Heterocyclus mit 1 bis 3 N- und/oder O-Atomen, der ein-, zwei- oder dreifach durch A, (CH₂)ₙOR¹¹ und/oder (CH₂)ₙN(R¹¹)₂ substituiert sein kann.

In einer weiteren Ausführungsform bedeutet Het besonders bevorzugt einen einkernigen aromatischen Heterocyclus mit 1 bis 2 N-Atomen, wie z.B. Pyridin oder Pyrimidin;
oder einen gesättigten Heterocyclus mit 1 bis 2 N-Atomen, wie z.B. Piperidin oder Piperazin, der ein- oder zweifach durch CH₂OH und/oder CH₂NA₂ substituiert sein kann.

X bedeutet vorzugsweise O oder S, ganz besonders bevorzugt O.
Y bedeutet vorzugsweise H oder A.

Die Verbindungen der Formel I können ein oder mehrere chirale Zentren besitzen und daher in verschiedenen stereoisomeren Formen vorkommen. Die Formel I umschließt alle diese Formen.

Die erfindungsgemäßen Verbindungen und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den erfindungsgemäßen Verbindungen umsetzt.

Die Ausgangsverbindungen sind in der Regel bekannt. Sind sie neu, so können sie aber nach an sich bekannten Methoden hergestellt werden.

Verbindungen der Formel I können vorzugsweise erhalten werden, indem man eine Verbindung der Formel II mit einem Hydrazid der Formel III umsetzt.

Die Umsetzung erfolgt nach Methoden, die dem Fachmann bekannt sind. Zunächst erfolgt Reaktion in einem geeigneten Lösungsmittel.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chlorform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel.
Als Lösungsmittel besonders bevorzugt sind Alkohole, wie z.B. Isopropanol oder Ethanol.

Die Reaktionszeit liegt je nach den angewendeten Bedingungen zwischen einigen Minuten und 14 Tagen, die Reaktionstemperatur zwischen etwa -30° und 140°, normalerweise zwischen -10° und 130°, insbesondere zwischen etwa 30° und etwa 125°.

In der so erhaltenen Verbindung der Formel I 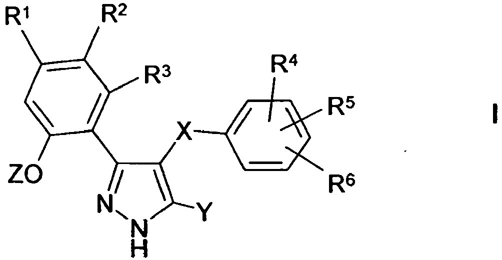 worin Z H oder Methyl bedeutet,
erfolgt gegebenenfalls die Etherspaltung nach Methoden, die dem Fachmann bekannt sind.
Die Reaktion erfolgt in einem geeigneten Lösungsmittel, wie oben angegeben, vorzugsweise durch Zugabe von Bortribromid.
Die Reaktion erfolgt besonders bevorzugt in Dichlormethan bei einer Reaktionstemperatur zwischen etwa -30° und 50°, normalerweise zwischen -20° und 20°, insbesondere zwischen etwa -15° und etwa 0°.

Man gelangt so zu Verbindungen der Formel I, worin Z H bedeutet.

Es ist ferner möglich, eine Verbindung der Formel I in eine andere Verbindung der Formel I umzuwandeln, indem man einen oder mehrere Rest(e) R¹, R², R³, R⁴, R⁵ und/oder R⁶ in einen oder mehrere andere Reste R¹, R², R³, R⁴, R⁵ und/oder R⁶ umwandelt, z.B. indem man Nitrogruppen, beispielsweise durch Hydrierung an Raney-Nickel oder Pd-Kohle in einem inerten Lösungsmittel wie Methanol oder Ethanol, zu Aminogruppen reduziert und/oder
eine Estergruppe in eine Carboxygruppe umwandelt und/oder eine Aminogruppe durch reduktive Aminierung in ein alkyliertes Amin umwandelt und/oder
Carboxygruppen durch Umsetzung mit Alkoholen verestert und/oder Säurechloride durch Umsetzung mit einem Amin in ein Säureamid überführt.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und /oder in Gegenwart einer Base wie Triethylamin oder Pyridin bei Temperaturen zwischen -60 und +30°.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der Verbindungen der Formel I gemäß Anspruch 1 werden größtenteils konventionell hergestellt. Sofern die Verbindung der Formel I gemäß Anspruch 1 eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methytglutamin. Die Aluminiumsalze der Verbindungen der Formel I gemäß Anspruch 1 zählen ebenfalls dazu. Bei bestimmten Verbindungen der Formel I gemäß Anspruch 1 lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der Verbindungen der Formel I die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der Verbindungen der Formel I gemäß Anspruch 1, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propyl-amin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der Formel I gemäß Anspruch 1 werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen.

Wie erwähnt werden die pharmazeutisch unbedenklichen Basenadditionssalze der Verbindungen der Formel I gemäß Anspruch 1 mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine Verbindung der Formel I gemäß Anspruch 1 in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Erfindungsgemäße Verbindungen der Formel I gemäß Anspruch 1 können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der Verbindungen der Formel I gemäß Anspruch 1 unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Gegenstand der Erfindung ist ferner die Verwendung der Verbindungen und/oder ihrer physiologisch unbedenklichen Salze zur Herstellung eines Arzneimittels (pharmazeutische Zubereitung), insbesondere auf nichtchemischem Wege. Hierbei können sie zusammen mit mindestens einem festen, flüssigen und/oder halbflüssigen Träger- oder Hilfsstoff und gegebenenfalls in Kombination mit einem oder mehreren weiteren Wirkstoffen in eine geeignete Dosierungsform gebracht werden.

Gegenstand der Erfindung sind ferner Arzneimittel, enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, sowie gegebenenfalls Träger- und/oder Hilfsstoffe.

Gegenstand der Eerfindung sind insbesondere solche Arzneimittel, enthaltend mindestens eine Verbindung, ausgewählt aus der Gruppe
3-(2,4-Dihydroxy-phenyl)-4-(4-fluor-phenoxy)-1*H*-pyrazol,
3-(2,4-Dihydroxy-5-ethyl-phenyl)-4-(4-fluor-phenoxy)-5-methyl-1*H*-pyrazol,
3-(2,4-Dihydroxy-phenyl)-4-(4-fluor-phenoxy)-5-trifluormethyl-1*H*-pyrazol,
3-(2,4-Dihydroxy-phenyl)-4-(4-ethoxy-phenoxy)-1*H*-pyrazol,
3-(2,4-Dihydroxy-phenyl)-4-phenoxy-1*H*-pyrazol,
3-(2,4-Dihydroxy-phenyl)-4-(4-methyl-phenoxy)-1*H*-pyrazol,
3-(2,4-Dihydroxy-phenyl)-4-(3-methyl-phenoxy)-1*H*-pyrazol,
3-(2,4-Dihydroxy-phenyl)-4-(2-methyl-phenoxy)-1*H*-pyrazol,
3-(2,4-Dihydroxy-phenyl)-4-(2-amino-phenoxy)-1*H*-pyrazol,
3-(2,4-Dihydroxy-phenyl)-4-(3-amino-phenoxy)-1*H*-pyrazol,
3-(2,4-Dihydroxy-phenyl)-4-(4-amino-phenoxy)-1*H*-pyrazol,
3-(2,4-Dihydroxy-phenyl)-4-(3-fluor-phenoxy)-1*H*-pyrazol,
3-(2,4-Dihydroxy-phenyl)-4-(2-fluor-phenoxy)-1*H*-pyrazol,
3-(2,4-Dihydroxy-phenyl)-4-(4-cyan-phenoxy)-1*H*-pyrazol,
3-(2,4-Dihydroxy-phenyl)-4-(2-cyan-phenoxy)-1*H*-pyrazol,
3-(2,4-Dihydroxy-phenyl)-4-(3-cyan-phenoxy)-1*H*-pyrazol,
3-(2,4-Dihydroxy-phenyl)-4-(2,4-dimethyl-phenoxy)-1*H*-pyrazol,
3-(2,4-Dihydroxy-phenyl)-4-(2,5-dimethyl-phenoxy)-1*H*-pyrazol,
3-(2,4-Dihydroxy-phenyl)-4-(2,6-dimethyl-phenoxy)-1*H*-pyrazol,
3-(2,4-Dihydroxy-phenyl)-4-(3,4-dimethyl-phenoxy)-1*H*-pyrazol,
3-(2,4-Dihydroxy-phenyl)-4-(2-ethyl-phenoxy)-1*H-*pyrazol,
3-(2,4-Dihydroxy-phenyl)-4-(3-ethyl-phenoxy)-1*H-*pyrazol,
3-(2,4-Dihydroxy-phenyl)-4-(3,5-dimethyl-phenoxy)-1*H*-pyrazol,
3-(2,4-Dihydroxy-phenyl)-4-(4-ethyl-phenoxy)-1*H*-pyrazol,
3-(2,4-Dihydroxy-phenyl)-4-(2,3-dimethyl-phenoxy)-1*H*-pyrazol,
3-(2,4-Dihydroxy-phenyl)-4-(4-amino-3-methyl-phenoxy)-1*H*-pyrazol,
3-(2,4-Dihydroxy-phenyl)-4-(2-amino-5-methyl-phenoxy)-1*H-*pyrazol,
3-(2,4-Dihydroxy-phenyl)-4-(2-amino-4-methyl-phenoxy)-1*H*-pyrazol,
3-(2,4-Dihydroxy-phenyl)-4-(3-amino-6-methyl-phenoxy)-1*H*-pyrazol,
3-(2,4-Dihydroxy-phenyl)-4-(4-amino-3-fluor-phenoxy)-1*H*-pyrazol,
3-(2,4-Dihydroxy-phenyl)-4-(2-chlor-phenoxy)-1*H*-pyrazol,
3-(2,4-Dihydroxy-phenyl)-4-(4-chlor-phenoxy)-1*H*-pyrazol,
3-(2,4-Dihydroxy-phenyl)-4-(3-chlor-phenoxy)-1*H*-pyrazol,
3-(2,4-Dihydroxy-phenyl)-4-(3,4-difluor-phenoxy)-1*H*-pyrazol,
3-(2,4-Dihydroxy-phenyl)-4-(2,3-difluor-phenoxy)-1*H*-pyrazol,
3-(2,4-Dihydroxy-phenyl)-4-(3,5-difluor-phenoxy)-1*H-*pyrazol,
3-(2,4-Dihydroxy-phenyl)-4-(2,4-difluor-phenoxy)-1*H*-pyrazol,
3-(2,4-Dihydroxy-phenyl)-4-(2,6-difluor-phenoxy)-1*H*-pyrazol,
3-(2,4-Dihydroxy-phenyl)-4-(indol-6-yloxy)-1*H*-pyrazol,
3-(2,4-Dihydroxy-phenyl)-4-(indol-4-yloxy)-1*H*-pyrazol,
3-(2,4-Dihydroxy-phenyl)-4-(indol-5-yloxy)-1*H*-pyrazol,
3-(2,4-Dihydroxy-phenyl)-4-(4-cyan-3-fluor-phenoxy)-1*H*-pyrazol,
3-(2,4-Dihydroxy-phenyl)-4-(2-aminocarbonyl-phenoxy)-1*H*-pyrazol,
3-(2,4-Dihydroxy-phenyl)-4-(4-aminocarbonyl-phenoxy)-1*H*-pyrazol,
3-(2,4-Dihydroxy-phenyl)-4-(4-acetamido-phenoxy)-1H-pyrazol,
3-(2,4-Dihydroxy-phenyl)-4-(2-acetamido-phenoxy)-1*H*-pyrazol,
3-(2,4-Dihydroxy-phenyl)-4-(3-acetamido-phenoxy)-1*H*-pyrazol,
3-(2,4-Dihydroxy-phenyl)-4-(3-ureido-phenoxy)-1*H-*pyrazol,
3-(2,4-Dihydroxy-phenyl)-4-(3-dimethylamino-phenoxy)-1*H*-pyrazol,
3-(2,4-Dihydroxy-phenyl)-4-(3-methoxycarbonyl-phenoxy)-1*H*-pyrazol,
3-(2,4-Dihydroxy-phenyl)-4-(2-methoxycarbonyl-phenoxy)-1*H*-pyrazol,
3-(2,4-Dihydroxy-phenyl)-4-[(4-fluor-phenyl)-sulfanyl]-1*H*-pyrazol,
3-(2,4-Dihydroxy-5-brom-phenyl)-4-(3-methyl-phenoxy)-1*H-*pyrazol,
3-(2,4-Dihydroxy-5-brom-phenyl)-4-(2,3-dibrom-phenoxy)-1*H*-pyrazol,
3-(2,4-Dihydroxy-5-brom-phenyl)-4-(4-fluor-phenoxy)-1*H*-pyrazol,
3-(2,4-Dihydroxy-5-brom-phenyl)-4-(3-chlor-phenoxy)-1*H-*pyrazol,
3-(2,4-Dihydroxy-5-brom-phenyl)-4-(4-chlor-phenoxy)-1*H-*pyrazol,
3-(2,4-Dihydroxy-5-brom-phenyl)-4-(4-ethyl-phenoxy)-1*H*-pyrazol,
3-(2,4-Dihydroxy-5-brom-phenyl)-4-(2,5-dimethyl-phenoxy)-1*H*-pyrazol,
3-(2,4-Dihydroxy-5-brom-phenyl)-4-(4-cyan-phenoxy)-1*H*-pyrazol,
3-(2,4-Dimethoxy-phenyl)-4-[(4-fluor-phenyl)-sulfanyl]-1*H*-pyrazol,
3-[2,4-Dihydroxy-5-(3-hydroxymethyl-piperidin-1-sulfonyl)-phenyl]-4-(2-chlor-phenoxy)-1*H*-pyrazol,
3-[2,4-Dihydroxy-5-diethylsulfamoyl-phenyl]-4-(2-chlor-4-diethylsulfamoylphenoxy)-1*H*-pyrazol,
3-[2,4-Dihydroxy-5-(pyridin-4-yl-sulfamoyl)-phenyl]-4-(2-chlor-phenoxy)-1*H-*pyrazol,
3-[2,4-Dihydroxy-5-(pyridin-2-yl-sulfamoyl)-phenyl]-4-(2-chlor-phenoxy)-1*H-*pyrazol,
3-[2,4-Dihydroxy-5-(pyridin-3-yl-sulfamoyl)-phenyl]-4-(2-chlor-phenoxy)-1*H-*pyrazol,
3-[2,4-Dihydroxy-5-(benzyl-sulfamoyl)-phenyl]-4-(2-chlor-phenoxy)-1*H-*pyrazol,
3-[2,4-Dihydroxy-5-(4-fluorphenyl-sulfamoyl)-phenyl]-4-(2-chlor-phenoxy)-1*H*-pyrazol,
3-[2,4-Dihydroxy-5-(3-fluorphenyl-sulfamoyl)-phenyl]-4-(2-chlor-phenoxy)-1*H*-pyrazol,
3-[2,4-Dihydroxy-5-(2-fluorphenyl-sulfamoyl)-phenyl]-4-(2-chlor-phenoxy)-1*H-*pyrazol,
3-[2,4-Dihydroxy-5-(phenyl-sulfamoyl)-phenyl]-4-(2-chlor-phenoxy)-1*H-*pyrazol,
3-[2,4-Dihydroxy-5-(2-diethylaminomethyl-piperidin-1-sulfonyl)-phenyl]-4-(2-chlor-phenoxy)-1*H*-pyrazol,
3-[2,4-Dihydroxy-5-(4-methyl-piperazin-1-sulfonyl)-phenyl]-4-(2-chlor-phenoxy)-1*H*-pyrazol,
3-[2,4-Dihydroxy-5-(piperidin-1-sulfonyl)-phenyl]-4-(2-chlor-phenoxy)-1*H-*pyrazol,
3-[2,4-Dihydroxy-5-(diethyl-sulfamoyl)-phenyl]-4-(2-chlor-phenoxy)-1*H-*pyrazol,
sowie ihre pharmazeutisch verwendbaren Salze, Solvate, Tautomere und Stereoisomere, einschließlich deren Mischungen in allen Verhältnissen.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,1 mg bis 3 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder Öl-in-Wasser-Flüssigemulsionen oder Wasser-in-Öl-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glycerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern.

Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs- oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymer-material und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte Isostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die Verbindungen der Formel I gemäß Anspruch 1 sowie Salze und Solvate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die Verbindungen der Formel I gemäß Anspruch 1 sowie die Salze und Solvate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektions-lösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer Verbindung der Formel I gemäß Anspruch 1 hängt von einer Reihe von Faktoren ab, einschließlich z.B. dem Alter und Gewicht des Menschen oder Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der Verbindung der Formel I gemäß Anspruch 1 *per se* bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine Verbindung der Formel I gemäß Anspruch 1 und/oder ihre pharmazeutisch verwendbaren Salze, Solvate und Stereoisomere, einschließlich deren Mischungen in allen Verhältnissen, und mindestens einen weiteren Arzneimittelwirkstoff.

Als weitere Arzneimittelwirkstoffe sind Chemotherapeutika bevorzugt, insbesondere solche, die Angiogenese hemmen und dadurch das Wachstum und die Verbreitung von Tumorzellen inhibieren; bevorzugt sind dabei VEGF-Rezeptorinhibitoren, beinhaltend Robozyme und Antisense, die auf VEGF-Rezeptoren gerichtet sind, sowie Angiostatin und Endostatin.

Beispiele antineoplastischer Agenzien, die in Kombination mit den erfindungsgemäßen Verbindungen verwendet werden können, beinhalten im allgemeinen alkylierende Agenzien, Antimetaboliten; Epidophyllotoxin; ein antineoplastisches Enzym; einen Topoisomerase-Inhibitor; Procarbazin; Mitoxantron oder Platin-Koordinationskomplexe.

Antineoplastische Agenzien sind vorzugsweise ausgewählt aus den folgenden Klassen:
Anthracycline, Vinca-Arzneistoffe, Mitomycine, Bleomycine, cytotoxische Nukleoside, Epothilone, Discodermolide, Pteridine, Diynene und Podophyllotoxine.
Besonders bevorzugt sind in den genanten Klassen z.B. Carminomycin, Daunorubicin, Aminopterin, Methotrexat, Methopterin, Dichlormethotrexat, Mitomycin C, Porfiromycin, 5-Fluoruracil, 5-Fluordeoxyuridin Monophosphat, Cytarabine, 5-Azacytidin, Thioguanin, Azathioprine, Adenosin, Pentostatin, Erythrohydroxynonyladenin, Cladribine, 6-Mercaptopurin, Gemcitabine, Cytosinarabinosid, Podophyllotoxin oder Podophyllotoxinderivate, wie z.B. Etoposide, Etoposide Phosphat oder Teniposide, Melphalan, Vinblastine, Vinorelbine, Vincristine, Leurosidine, Vindesine, Leurosine, Docetaxel und Paclitaxel. Andere bevorzugte antineoplastische Agenzien sind ausgewählt aus der Gruppe Discodermolide, Epothilone D, Estramustine, Carboplatin, Cisplatin, Oxaliplatin, Cyclophosphamid, Bleomycin, Gemcitabine, Ifosamide, Melphalan, Hexamethylmelamin, Thiotepa, Idatrexate, Trimetrexate, Dacarbazine, L-Asparaginase, Camptothecin, CPT-11, Topotecan, Arabinosyl-Cytosin, Bicalutamide, Flutamide, Leuprolide, Pyridobenzoindolderivate, Interferone und Interleukine.

Als weitere Arzneimittelwirkstoffe sind Antibiotica bevorzugt. Bevorzugte Antibiotica sind ausgewählt aus der Gruppe
Dactinomycin, Daunorubicin, Idarubicin, Epirubicin, Mitoxantrone, Bleomycin, Plicamycin, Mitomycin.

Als weitere Arzneimittelwirkstoffe sind Enzyminhibitoren bevorzugt. Bevorzugte Enzyminhibitoren sind ausgewählt aus der Gruppe der Histon-Deacetylierungs-Inhibitoren (z.B. suberoylanilide hydroxamic acid [SAHA]) und der Tyrosinkinase-Inhibitoren (z.B. ZD 1839 [Iressa]).

Als weitere Arzneimittelwirkstoffe sind Nuclear-Export-Inhibitoren bevorzugt. Nuclear-Export-Inhibitoren verhindern die Ausschleusung von Biopolymeren (z.B. RNA) aus dem Zellkern. Bevorzugte Nuclear-Export-Inhibitoren sind ausgewählt aus der Gruppe Callystatin, Leptomycin B, Ratjadone.

Als weitere Arzneimittelwirkstoffe sind Nuclear-Export-Inhibitoren bevorzugt. Nuclear-Export-Inhibitoren verhindern die Ausschleusung von Biopolymeren (z.B. RNA) aus dem Zellkern. Bevorzugte Nuclear-Export-Inhibitoren sind ausgewählt aus der Gruppe Callystatin, Leptomycin B, Ratjadone.

Als weitere Arzneimittelwirkstoffe sind Immunsuppressiva bevorzugt. Bevorzugte Immunsuppressiva sind ausgewählt aus der Gruppe Rapamycin, CCI-779 (Wyeth), RAD001 (Novartis), AP23573 (Ariad Pharmaceuticals).

Gegenstand der Erfindung ist auch ein Set (Kit), bestehend aus getrennten Packungen von
(a) einer wirksamen Menge an einer Verbindung der Formel I gemäß Anspruch 1 und/oder ihrer pharmazeutisch verwendbaren Salze, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer Verbindung der Formel I gemäß Anspruch 1 und/oder ihrer pharmazeutisch verwendbaren Salze, Solvate und Stereoisomere, einschließlich deren Mischungen in allen Verhältnissen, und einer wirksamen Menge eines weiteren Arzneimittelwirkstoffs gelöst oder in lyophylisierter Form vorliegt.

### VERWENDUNG

Die vorliegenden Verbindungen eignen sich als pharmazeutische Wirkstoffe für Säugetiere, insbesondere für den Menschen, bei der Behandlung von Krankheiten, bei denen HSP90 eine Rolle spielt. Gegenstand der Erfindung ist somit die Verwendung von Verbindungen der Formel I gemäß Anspruch 1, sowie ihrer pharmazeutisch verwendbaren Salze, Solvate und Stereoisomere, einschließlich deren Mischungen in allen Verhältnissen, zur Herstellung eines Arzneimittels zur Behandlung von Krankheiten, bei denen die Hemmung, Regulierung und/oder Modulation von HSP90 eine Rolle spielt.

Die vorliegende Erfindung umfasst die Verwendung der Verbindungen der Formel I gemäß Anspruch 1 und/oder ihre physiologisch unbedenklichen Salze und Solvate zur Herstellung eines Arzneimittels zur Behandlung von Tumorerkrankungen, wie z.B. Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung; viralen Erkrankungen, wobei das virale Pathogen ausgewählt ist aus der Gruppe, bestehend aus Hepatitis Typ A, Hepatitis Typ B, Hepatitis Typ C, Influenza, Varicella, Adenovirus, Herpes-Simplex Typ 1 (HSV-I), Herpes Simplex Typ II (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masernvirus, Rötelnvirus, Poliovirus, menschliches Immunschwächevirus Typ I (HIV-I) und menschliches Immunschwächevirus Typ II (HIV-II); zur Immunsuppression bei Transplantationen; entzündungsbedingten Erkrankungen, wie Rheumatoide Arthritis, Asthma, Multiple Sklerose, Typ 1 Diabetes, Lupus Erythematodes, Psoriasis und Inflammatory Bowel Disease; Zystische Fibrose; Erkrankungen im Zusammenhang mit Angiogenese wie z.B. diabetische Retinopathie, Hämangiome, Endometriose, Tumorangiogenese; infektiösen Erkrankungen; Autoimmunerkrankungen; Ischämie; Förderung der Nervenregeneration; fibrogenetischen Erkrankungen, wie z.B. Sklerodermie, Polymyositis, systemischer Lupus, Leberzirrhose, Keloidbildung, interstitielle Nephritis und pulmonare Fibrose;

Die Verbindungen der Formel I gemäß Anspruch 1 können insbesondere das Wachstum von Krebs, Tumorzellen und Tumormetastasen hemmen und sind deshalb für die Tumortherapie geeignet.

Die vorliegende Erfindung umfasst weiterhin die Verwendung der Verbindungen der Formel I gemäß Anspruch 1 und/oder ihre physiologisch unbedenklichen Salze und Solvate zur Herstellung eines Arzneimittels zum Schutz normaler Zellen gegen Toxizität, die durch Chemotherapie verursacht ist, sowie zur Behandlung von Krankheiten, wobei Proteinfehlfaltung oder Aggregation ein Hauptkausalfaktor ist, wie z.B. Skrapie, Creutzfeldt-Jakob-Krankheit, Huntington oder Alzheimer.

Die Erfindung betrifft auch die Verwendung der Verbindungen der Formel I gemäß Anspruch 1 und/oder ihre physiologisch unbedenklichen Salze und Solvate zur Herstellung eines Arzneimittels zur Behandlung von Krankheiten des Zentralnervensystems, von Herzkreislauferkrankungen und Kachexie.

Die Erfindung betrifft in einer weiteren Ausführungsform auch die Verwendung der Verbindungen der Formel I gemäß Anspruch 1 und/oder ihre physiologisch unbedenklichen Salze und Solvate zur Herstellung eines Arzneimittels zur HSP90-Modulation, wobei die modulierte biologische HSP90-Aktivität eine Immunreaktion in einem Individuum, Proteintransport vom endoplasmatischen Retikulum, Genesung vom hypoxischen/anoxischen Stress, Genesung von Unterernährung, Genesung von Hitzestress, oder Kombinationen davon, hervorruft, und/oder wobei die Störung eine Art Krebs ist, eine Infektionserkrankung, eine Störung, die mit einem gestörten Proteintransport vom endoplasmatischen Retikulum, einer Störung, die mit Ischämie / Reperfusion einhergeht, oder Kombinationen davon, wobei die die mit Ischämie / Reperfusion einhergehende Störung eine Folge von Herzstillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

Die Erfindung betrifft in einer weiteren Ausführungsform auch die Verwendung der Verbindungen der Formel I gemäß Anspruch 1 und/oder ihre physiologisch unbedenklichen Salze und Solvate zur Herstellung eines Arzneimittels zur Behandeln von Ischämie als Folge von Herz-stillstand, Asystole und verzögerten ventrikulären Arrythmien, Herzoperation, kardiopulmonärer Bypass-Operation, Organtransplantation, Rückenmarksverletzung, Kopftrauma, Schlaganfall, thromboembolischem Schlaganfall, hämorrhagischem Schlaganfall, cerebralem Vasospasmus, Hypotonie, Hypoglykämie, Status epilepticus, einem epileptischem Anfall, Angst, Schizophrenie, einer neurodegenerativen Störung, Alzheimer-Krankheit, Chorea Huntington, amyotropher lateraler Sklerose (ALS) oder Stress beim Neugeborenen ist.

### Testverfahren zur Messung von HSP90 Inhibitoren

Die Bindung von Geldanamycin oder 17- Allylamino-17-demethoxy-geldanamycin (17AAG) und deren kompetitive Hemmung an HSP90 kann benutzt werden, um die inhibitorische Aktivität der erfindungsgemäßen Verbindungen zu bestimmen (Carreras et al. 2003, Chiosis et al. 2002). Im speziellen Fall wird ein Radioligand-Filterbindungstest verwendet. Als Radioligand wird dabei mit Tritium markiertes 17-Allylamino-geldanamycin, [3H]17AAG, verwendet. Dieser Filter-Bindungstest erlaubt eine gezielte Suche nach Inhibitoren, die mit der ATP-Bindestelle interferieren.

### Material

Rekombinantes humanes HSP90α (E. coli exprimiert, 95% Reinheit); [3H]17AAG (17-Allylamino-geldanamycin, [allylamino-2,3-³H. Spezifische Aktivität: 1,11x10¹² Bq/mmol (Moravek, MT-1717); HEPES Filterpuffer (50 mM HEPES, pH 7,0, 5mM MgCl2, BSA 0.01%) Multiscreen-FB (1µm) Filterplatte (Millipore, MAFBNOB 50).

### Methode

Die 96 weil Mikrotiter-Filterplatten werden zunächst gewässert und mit 0,1% Polyethylenimin beschichtet.

Der Test wird unter folgenden Bedingungen durchgeführt:
Reaktionstemperatur 22 °C
Reaktionszeit: 30 min., Schütteln bei 800 upm
Testvolumen: 50 µl

### Endkonzentrationen:

50 mM HEPES-HCl, pH7,0, 5 mM MgCl2, 0,01 % (w/v) BSA
HSP90: 1,5 µg/assay
[3H]17AAG: 0,08 µM.

Am Ende der Reaktion wird der Überstand in der Filterplatte mit Hilfe eines Vakuum-Manifolds (Multiscreen Separation System, Millipore) abgesaugt und der Filter zweimal gewaschen.

Die Filterplatten werden dann in einem Beta-counter (Microbeta, Wallac) mit Szintillator (Microscint 20, Packard) gemessen.

Aus den "counts per minutes"-Werten wird "% der Kontrolle" ermittelt und daraus der IC-50 Wert einer Verbindung kalkuliert.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

### LC-MS Bedingungen

Hewlett Packard System der HP 1100 Serie mit den folgenden Merkmalen: Ionenquelle: Elektrospray (positive mode); Scan: 100-1000 m/z; Fragmentier-Spannung: 60 V; Gas-Temperatur: 300°C, DAD: 220 nm.

Flussrate: 2.4 ml/Min. Der verwendete Splitter reduzierte nach dem DAD die Flussrate für das MS auf 0,75ml/Min.
Säule: Chromolith SpeedROD RP-18e 50-4.6
Lösungsmittel: LiChrosolv-Qualität der Fa. Merck KGaA
Lösungsmittel A: H2O (0.01% TFA)
Lösungsmittel B: ACN (0.008% TFA)

### Gradient:

20% B → 100% B: 0 min bis 2.8 min
100% B: 2.8 min bis 3.3 min
100%B → 20%B: 3.3 min bis 4 min

Die in den nachfolgenden Beispielen angegebenen Retentionszeiten R_{f} [min] und M+H⁺-Daten MW sind die Meßergebnisse der LC-MS-Messungen.

### Beispiel 1

Herstellung von 3-(2,4-Dihydroxy-phenyl)-4-(4-fluor-phenoxy)-1H-pyrazol ("A1"):
1. Eine Lösung von 1,0 g 2,4-Dimethoxychloracetophenon, 530 mg 4-Fluorphenol, 100 mg Tetra-n-butylammoniumiodid und 1,0 g Kaliumcarbonat in 50 ml Acetonitril wird 1 Stunde unter Rückfluß erhitzt. Man arbeitet wie üblich auf und erhält 1,2 g 1-(2,4-Dimethoxy-phenyl)-2-(4-fluor-phenoxy)-ethanon ("1").
2. Eine Mischung von 200 mg "1" und 0,2 ml N,N-Dimethylformamid-dimethylacetal wird bei 160-180° 30 Minuten in der Mikrowelle bestrahlt. Man erhält 1-(2,4-Dimethoxy-phenyl)-3-dimethylamino-2-(4-fluor-phenoxy)-propenon 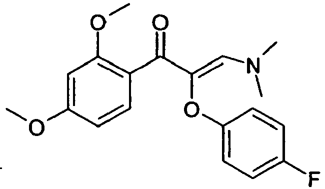 ("2"), das ohne weitere Aufreinigung umgesetzt wird.
3. Eine Lösung von 220 mg "2" und 100 µl Hydraziniumhydroxid in 2ml Ethanol wird bei 120° 30 Minuten in der Mikrowelle bestrahlt. Nach Aufreinigung mittels Flashchromatographie erhält man 109 mg 3-(2,4-Dimethoxy-phenyl)-4-(4-fluor-phenoxy)-1H-pyrazol ("3"), R_{f} 1,966; MW 314.
4. Eine Lösung von 14 mg "3" in 1 ml Dichlormethan wird unter Eiskühlung mit 50 µl Bortribromid versetzt und bei Raumtemperatur 50 Stunden nachgerührt. Nach Aufreinigung erhält man 3-(2,4-Dihydroxyphenyl)-4-(4-fluor-phenoxy)-1*H*-pyrazol ("A1"), R_{f} 1,026; MW 286.

Analog erhält man die nachstehenden Verbindungen
3-(2,4-Dihydroxy-5-ethyl-phenyl)-4-(4-fluor-phenoxy)-5-methyl-1 H-pyrazol,
3-(2,4-Dihydroxy-phenyl)-4-(4-fluor-phenoxy)-5-trifluormethyl-1*H-*pyrazol,
3-(2,4-Dihydroxy-phenyl)-4-(4-ethoxy-phenoxy)-1*H*-pyrazol,
3-(2,4-Dihydroxy-phenyl)-4-phenoxy-1*H*-pyrazol,
3-(2,4-Dihydroxy-phenyl)-4-(4-methyl-phenoxy)-1*H*-pyrazol, R_{f} 1,817; MW 282;
3-(2,4-Dihydroxy-phenyl)-4-(3-methyl-phenoxy)-1*H*-pyrazol, R_{f} 1,806; MW 282;
3-(2,4-Dihydroxy-phenyl)-4-(2-methyl-phenoxy)-1*H*-pyrazol, R_{f} 1,809;
3-(2,4-Dihydroxy-phenyl)-4-(2-amino-phenoxy)-1*H*-pyrazol,
3-(2,4-Dihydroxy-phenyl)-4-(3-amino-phenoxy)-1*H*-pyrazol,
3-(2,4-Dihydroxy-phenyl)-4-(4-amino-phenoxy)-1*H*-pyrazol,
3-(2,4-Dihydroxy-phenyl)-4-(3-fluor-phenoxy)-1*H*-pyrazol, R_{f} 1,716;
3-(2,4-Dihydroxy-phenyl)-4-(2-fluor-phenoxy)-1*H*-pyrazol, R_{f} 1,668;
3-(2,4-Dihydroxy-phenyl)-4-(4-cyan-phenoxy)-1*H*-pyrazol, R_{f} 1,532; MW 293;
3-(2,4-Dihydroxy-phenyl)-4-(2-cyan-phenoxy)-1*H*-pyrazol,
3-(2,4-Dihydroxy-phenyl)-4-(3-cyan-phenoxy)-1*H*-pyrazol,
3-(2,4-Dihydroxy-phenyl)-4-(2,4-dimethyl-phenoxy)-1*H*-pyrazol, R_{f} 1,965;
3-(2,4-Dihydroxy-phenyl)-4-(2,5-dimethyl-phenoxy)-1*H*-pyrazol, R_{f} 1,924; MW 296;
3-(2,4-Dihydroxy-phenyl)-4-(2,6-dimethyl-phenoxy)-1*H*-pyrazol, R_{f} 1,959; MW 296;
3-(2,4-Dihydroxy-phenyl)-4-(3,4-dimethyl-phenoxy)-1*H*-pyrazol, R_{f} 1,921;
3-(2,4-Dihydroxy-phenyl)-4-(2-ethyl-phenoxy)-1*H*-pyrazol, R_{f} 1,944;
3-(2,4-Dihydroxy-phenyl)-4-(3-ethyl-phenoxy)-1*H*-pyrazol, R_{f} 1,935;
3-(2,4-Dihydroxy-phenyl)-4-(3,5-dimethyl-phenoxy)-1*H*-pyrazol, R_{f} 1,929;
3-(2,4-Dihydroxy-phenyl)-4-(4-ethyl-phenoxy)-1*H*-pyrazol, R_{f} 1,954; MW 296;
3-(2,4-Dihydroxy-phenyl)-4-(2,3-dimethyl-phenoxy)-1*H*-pyrazol, R_{f} 1,913;
3-(2,4-Dihydroxy-phenyl)-4-(4-amino-3-methyl-phenoxy)-1*H*-pyrazol,
3-(2,4-Dihydroxy-phenyl)-4-(2-amino-5-methyl-phenoxy)-1*H*-pyrazol,
3-(2,4-Dihydroxy-phenyl)-4-(2-amino-4-methyl-phenoxy)-1*H*-pyrazol,
3-(2,4-Dihydroxy-phenyl)-4-(3-amino-6-methyl-phenoxy)-1*H*-pyrazol,
3-(2,4-Dihydroxy-phenyl)-4-(4-amino-3-fluor-phenoxy)-1*H*-pyrazol,
3-(2,4-Dihydroxy-phenyl)-4-(2-chlor-phenoxy)-1*H*-pyrazol, R_{f} 1,749;
3-(2,4-Dihydroxy-phenyl)-4-(4-chlor-phenoxy)-1H-pyrazol, R_{f} 1,832; MW 303;
3-(2,4-Dihydroxy-phenyl)-4-(3-chlor-phenoxy)-1*H*-pyrazol, R_{f} 1,816; MW 303;
3-(2,4-Dihydroxy-phenyl)-4-(3,4-difluor-phenoxy)-1*H*-pyrazol, R_{f} 1,728; MW 304;
3-(2,4-Dihydroxy-phenyl)-4-(2,3-difluor-phenoxy)-1*H*-pyrazol, R_{f} 1,704; MW 304;
3-(2,4-Dihydroxy-phenyl)-4-(3,5-difluor-phenoxy)-1*H*-pyrazol,
3-(2,4-Dihydroxy-phenyl)-4-(2,4-difluor-phenoxy)-1*H*-pyrazol, R_{f} 1,688;
3-(2,4-Dihydroxy-phenyl)-4-(2,6-difluor-phenoxy)-1*H*-pyrazol, R_{f} 1,625;
3-(2,4-Dihydroxy-phenyl)-4-(indol-6-yloxy)-1*H*-pyrazol,
3-(2,4-Dihydroxy-phenyl)-4-(indol-4-yloxy)-1*H*-pyrazol,
3-(2,4-Dihydroxy-phenyl)-4-(indol-5-yloxy)-1*H*-pyrazol,
3-(2,4-Dihydroxy-phenyl)-4-(4-cyan-3-fluor-phenoxy)-1*H*-pyrazol, R_{f} 1,853;
3-(2,4-Dihydroxy-phenyl)-4-(2-aminocarbonyl-phenoxy)-1*H*-pyrazol,
3-(2,4-Dihydroxy-phenyl)-4-(4-aminocarbonyl-phenoxy)-1*H*-pyrazol,
3-(2,4-Dihydroxy-phenyl)-4-(4-acetamido-phenoxy)-1*H*-pyrazol,
3-(2,4-Dihydroxy-phenyl)-4-(2-acetamido-phenoxy)-1*H*-pyrazol,
3-(2,4-Dihydroxy-phenyl)-4-(3-acetamido-phenoxy)-1*H*-pyrazol,
3-(2,4-Dihydroxy-phenyl)-4-(3-ureido-phenoxy)-1*H*-pyrazol,
3-(2,4-Dihydroxy-phenyl)-4-(3-dimethylamino-phenoxy)-1*H*-pyrazol, R_{f} 1;210;
3-(2,4-Dihydroxy-phenyl)-4-(3-methoxycarbonyl-phenoxy)-1*H*-pyrazol,
3-(2,4-Dihydroxy-phenyl)-4-(2-methoxycarbonyl-phenoxy)-1*H*-pyrazol,
3-(2,4-Dihydroxy-phenyl)-4-[(4-fluor-phenyl)-sulfanyl]-1*H*-pyrazol, R_{f} 1,877; MW 302;
3-(2,4-Dihydroxy-5-brom-phenyl)-4-(3-methyl-phenoxy)-1*H*-pyrazol, R_{f} 2,052; MW 361;
3-(2,4-Dihydroxy-5-brom-phenyl)-4-(2,3-dibrom-phenoxy)-1*H*-pyrazol, R_{f} 1,938; MW 383;
3-(2,4-Dihydroxy-5-brom-phenyl)-4-(4-fluor-phenoxy)-1*H*-pyrazol, R_{f} 1,704; MW 304;
3-(2,4-Dihydroxy-5-brom-phenyl)-4-(3-chlor-phenoxy)-1*H*-pyrazol, R_{f} 2,071; MW 382;
3-(2,4-Dihydroxy-5-brom-phenyl)-4-(4-chlor-phenoxy)-1*H*-pyrazol, R_{f} 2,091; MW 382;
3-(2,4-Dihydroxy-5-brom-phenyl)-4-(4-ethyl-phenoxy)-1*H*-pyrazol, R_{f} 2,185; MW 375;
3-(2,4-Dihydroxy-5-brom-phenyl)-4-(2,5-dimethyl-phenoxy)-1*H-*pyrazol, R_{f} 2,164; MW 375;
3-(2,4-Dihydroxy-5-brom-phenyl)-4-(4-cyan-phenoxy)-1*H*-pyrazol, R_{f} 1,790; MW 372;
3-(2,4-Dimethoxy-phenyl)-4-[(4-fluor-phenyl)-sulfanyl]-1*H*-pyrazol, R_{f} 2,342; MW 330;
3-(2,4-Dihydroxy-phenyl)-4-(3-carboxy-phenoxy)-1*H*-pyrazol, R_{f} 1,271;
3-(2,4-Dihydroxy-5-brom-phenyl)-4-(3-dimethylamino-phenoxy)-1*H-*pyrazol, R_{f} 1,434;
3-(2,4-Dihydroxy-5-brom-phenyl)-4-(2,6-difluor-phenoxy)-1*H*-pyrazol, R_{f} 1,890;
3-(2,4-Dihydroxy-5-brom-phenyl)-4-(2,4-difluor-phenoxy)-1*H*-pyrazol, R_{f} 1,939;
3-(2,4-Dihydroxy-5-brom-phenyl)-4-(2-chlor-phenoxy)-1*H*-pyrazol, R_{f} 1,997;
3-(2,4-Dihydroxy-5-brom-phenyl)-4-(2,3-dimethyl-phenoxy)-1*H-*pyrazol, R_{f} 2,154;
3-(2,4-Dihydroxy-5-brom-phenyl)-4-(3,5-dimethyl-phenoxy)-1*H-*pyrazol, R_{f} 2,169;
3-(2,4-Dihydroxy-5-brom-phenyl)-4-(3-ethyl-phenoxy)-1*H*-pyrazol, R_{f} 2,167;
3-(2,4-Dihydroxy-5-brom-phenyl)-4-(2-ethyl-phenoxy)-1*H*-pyrazol, R_{f} 2,177;
3-(2,4-Dihydroxy-5-brom-phenyl)-4-(3,4-dimethyl-phenoxy)-1*H-*pyrazol, R_{f} 2,068;
3-(2,4-Dihydroxy-5-brom-phenyl)-4-(2,4-dimethyl-phenoxy)-1*H-*pyrazol, R_{f} 2,321;
3-(2,4-Dihydroxy-5-brom-phenyl)-4-(2-fluor-phenoxy)-1*H*-pyrazol, R_{f} 1,918;
3-(2,4-Dihydroxy-5-brom-phenyl)-4-(2-methyl-phenoxy)-1*H*-pyrazol, R_{f} 2,057.

### Beispiel 2

### Herstellung von 3-(2,4-Dihydroxy-phenyl)-4-phenoxy-1H-pyrazol

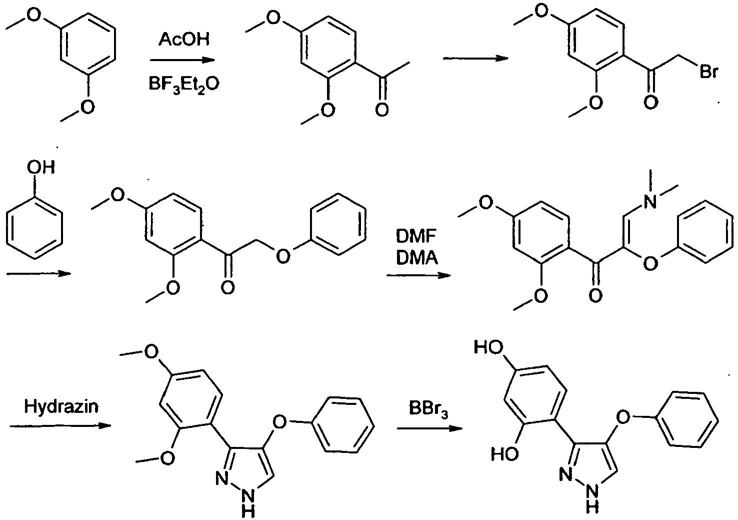

### Beispiel 3

### Herstellung von 3-[2,4-Dihydroxy-5-(3-hydroxymethyl-piperidin-1-sulfonyl)-phenyl]-4-(2-chlor-phenoxy)-1H-pyrazol:

3.1 Analog Beispiel 1, Stufe 2 erhält man die Verbindung 1-(2,4-Dimethoxy-phenyl)-3-dimethylamino-2-(2-chlor-phenoxy)-propenon 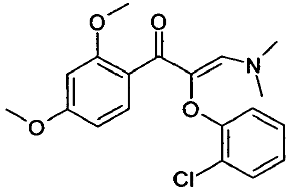
3.2 Analog Beispiel 1 Stufe 3 erhält man daraus durch Umsetzung mit Hydrazinumhydroxid die Verbindung 3-(2,4-Dimethoxy-phenyl)-4-(2-chlor-phenoxy)-1*H*-pyrazol.
3.3 Durch Umsetzung von 3-(2,4-Dimethoxy-phenyl)-4-(2-chlor-phenoxy)-1H-pyrazol mit Chlorsulfonsäure in Dichlormethan erhält man unter Standardbedingungen die Verbindung 3-(2,4-Dimethoxy-5-sulfo-phenyl)-4-(2-chlor-phenoxy)-1*H*-pyrazol.
3.4 Durch Umsetzung von 3-(2,4-Dimethoxy-5-sulfo-phenyl)-4-(2-chlor-phenoxy)-1H-pyrazol mit Thionylchlorid und einem Äquivalent DMF erhält man nach 4 Stunden Erhitzen und üblicher Aufarbeitung die Verbindung 3-(2,4-Dimethoxy-5-chlorsulfonyl-phenyl)-4-(2-chlor-phenoxy)-1*H*-pyrazol.
3.5 Eine Lösung von 44,96 µl 3-(Hydroxymethyl)-piperidin und 82,9 mg Pyridin in 4 ml Dichlormethan wird mit 150 mg 3-(2,4-Dimethoxy-5-chlorsulfonyl-phenyl)-4-(2-chlor-phenoxy)-1*H-*pyrazol versetzt und 16 Stunden gerührt. Nach üblicher Aufarbeitung erhält man in quantitativer Ausbeute die Verbindung 3-[2,4-Dimethoxy-5-(3-hydroxymethyl-piperidin-1-sulfonyl)-phenyl]-4-(2-chlor-phenoxy)-1*H*-pyrazol.
3.6 Analog Beispiel 1 Stufe 4 erhält man daraus durch Etherspaltung die Verbindung 3-[2,4-Dihydroxy-5-(3-hydroxymethyl-piperidin-1-sulfonyl)-phenyl]-4-(2-chlor-phenoxy)-1*H*-pyrazol, R_{f} 1,526; MW 480.94

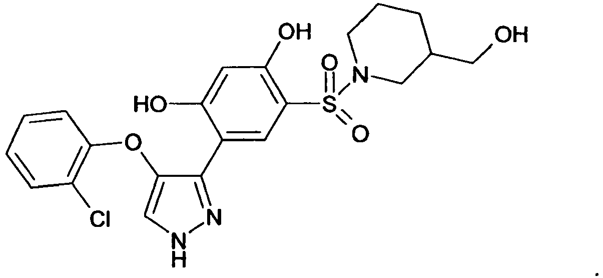

### Beispiel 4

### Herstellung von 3-[2,4-Dihydroxy-5-diethylsulfamoyl-phenyl]-4-(2-chlor-4-diethylsulfamoyl-phenoxy)-1H-pyrazol:

4.1 Durch Umsetzung von 3-(2,4-Dimethoxy-phenyl)-4-(2-chlor-phenoxy)-1H-pyrazol mit Essigsäureanhydrid erhält man unter Standardbedingungen die Verbindungen 1-Acetyl-3-(2,4-dimethoxy-phenyl)-4-(2-chlor-phenoxy)-1 H-pyrazol.
4.2 Eine Lösung von 112 mg 1-Acetyl-3-(2,4-dimethoxy-phenyl)-4-(2-chlor-phenoxy)-1*H*-pyrazol in 1 ml Dichlormethan wird zu 1 ml gekühlter Chlorsulfonsäure getropft. Man rührt eine Stunde unter Kühlung und läßt dann auf Raumtemperatur erwärmen. Man tropft alles auf Eiswasser, arbeitet wie üblich auf und erhält 133 mg 1-Acetyl-3-[2,4-dihydroxy-5-chlorsulfonyl-phenyl]-4-(2-chlor-4-chlorsulfonyl-phenoxy)-1*H*-pyrazol.
4.3 Eine Mischung von 133 mg 1-Acetyl-3-[2,4-dihydroxy-5-chlorsulfonyl-phenyl]-4-(2-chlor-4-chlorsulfonyl-phenoxy)-1*H*-pyrazol, 200 µl Diethylamin und 3 ml THF wird 4 Stunden bei Raumtemperatur gerührt. Das Lösungsmittel wird entfernt und der Rückstand wird flashchromatographiert (ISCO Companion^{®}). Man erhält 85 mg 3-[2,4-Dimethoxy-5-diethylsulfamoyl-phenyl]-4-(2-chlor-4-diethylsulfamoyl-phenoxy)-1*H*-pyrazol.
4.4 Analog Beispiel 1 Stufe 4 erhält man daraus durch Etherspaltung die Verbindung 3-[2,4-Dihydroxy-5-diethylsulfamoyl-phenyl]-4-(2-chlor-4-diethylsulfamoyl-phenoxy)-1*H*-pyrazol, R_{f} 1,977; MW 574.09

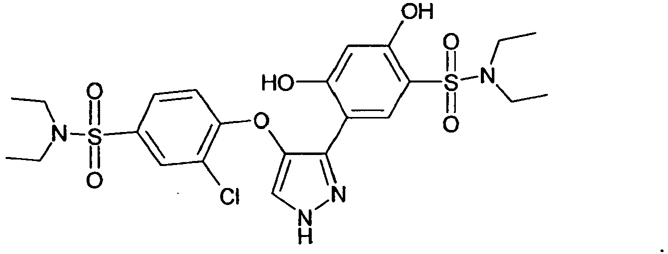

### Beispiel 5

### Herstellung von 3-[2,4-Dihydroxy-5-(pyridin-4-yl-sulfamoyl)-phenyl]-4-(2-chlor-phenoxy)-1H-pyrazol:

Analog Beispiel 3.5 erhält man durch Umsetzung von 3-(2,4-Dimethoxy-5-chlorsulfonyl-phenyl)-4-(2-chlor-phenoxy)-1*H*-pyrazol und 4-Aminopyridin die Verbindung 3-[2,4-Dimethoxy-5-(pyridin-4-yl-sulfamoyl)-phenyl]-4-(2-chlor-phenoxy)-1*H*-pyrazol und daraus durch Etherspaltung die Verbindung 3-[2,4-Dihydroxy-5-(pyridin-4-yl-sulfamoyl)-phenyl]-4-(2-chlor-phenoxy)-1*H*-pyrazol

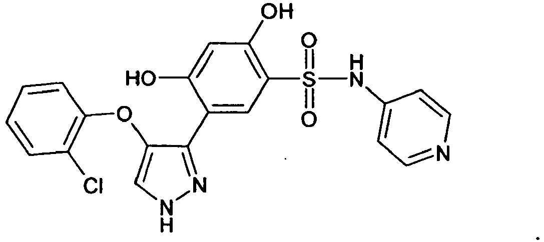

Analog erhält man die Verbindungen
3-[2,4-Dihydroxy-5-(pyridin-2-yl-sulfamoyl)-phenyl]-4-(2-chlor-phenoxy)-1*H*-pyrazol, R_{f} 1,408; MW 459.88;
3-[2,4-Dihydroxy-5-(pyridin-3-yl-sulfamoyl)-phenyl]-4-(2-chlor-phenoxy)-1*H*-pyrazol, R_{f} 1,209; MW 459.88;
3-[2,4-Dihydroxy-5-(benzyl-sulfamoyl)-phenyl]-4-(2-chlor-phenoxy)-1*H*-pyrazol, R_{f} 1,735; MW 472.92;
3-[2,4-Dihydroxy-5-(4-fluorphenyl-sulfamoyl)-phenyl]-4-(2-chlor-phenoxy)-1*H*-pyrazol, R_{f} 1,735; MW 476.88;
3-[2,4-Dihydroxy-5-(3-fluorphenyl-sulfamoyl)-phenyl]-4-(2-chlor-phenoxy)-1H-pyrazol, R_{f} 1,763; MW 476.88;
3-[2,4-Dihydroxy-5-(2-fluorphenyl-sulfamoyl)-phenyl]-4-(2-chlor-phenoxy)-1*H*-pyrazol, R_{f} 1,748; MW 476.88;
3-[2,4-Dihydroxy-5-(phenyl-sulfamoyl)-phenyl]-4-(2-chlor-phenoxy)-1*H*-pyrazol, R_{f} 1,704; MW 458.89;
3-[2,4-Dihydroxy-5-(2-diethylaminomethyl-piperidin-1-sulfonyl)-phenyl]-4-(2-chlor-phenoxy)-1*H*-pyrazol, R_{f} 1,154; MW 536.06;
3-[2,4-Dihydroxy-5-(4-methyl-piperazin-1-sulfonyl)-phenyl]-4-(2-chlor-phenoxy)-1*H*-pyrazol, R_{f} 0,995; MW 465,93;
3-[2,4-Dihydroxy-5-(piperidin-1-sulfonyl)-phenyl]-4-(2-chlor-phenoxy)-1*H*-pyrazol, R_{f} 2,002; MW 450,91;
3-[2,4-Dihydroxy-5-(diethyl-sulfamoyl)-phenyl]-4-(2-chlor-phenoxy)-1*H*-pyrazol, R_{f} 1,840; MW 438,90.

Die nachfolgenden Beispiele betreffen pharmazeutische Zubereitungen:

### Beispiel A: Injektionsgläser

Eine Lösung von 100 g eines erfindungsgemäßen Wirkstoffes und 5 g Dinatriumhydrogenphosphat wird in 3 l zweifach destilliertem Wasser mit 2 n Salzsäure auf pH 6,5 eingestellt, steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines erfindungsgemäßen Wirkstoffes mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines erfindungsgemäßen Wirkstoffes, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 l auf und sterilisiert durch Bestrahlung. Diese Lösung kann in Form von Augentropfen verwendet werden.

### Beispiel D: Salbe

Man mischt 500 mg eines erfindungsgemäßen Wirkstoffes mit 99,5 g Vaseline unter aseptischen Bedingungen.

### Beispiel E: Tabletten

Ein Gemisch von 1 kg Wirkstoff, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff werden in üblicher Weise in Hartgelatine kapseln gefüllt, so daß jede Kapsel 20 mg des Wirkstoffs enthält.

### Beispiel H: Ampullen

Eine Lösung von 1 kg eines erfindungsgemäßen Wirkstoffes in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen ausgewählt aus der Gruppe
3-(2,4-Dihydroxy-phenyl)-4-(4-methyl-phenoxy)-1*H*-pyrazol,
3-(2,4-Dihydroxy-phenyl)-4-(3-methyl-phenoxy)-1*H*-pyrazol,
3-(2,4-Dihydroxy-phenyl)-4-(2-methyl-phenoxy)-1*H*-pyrazol,
3-(2,4-Dihydroxy-phenyl)-4-(2-amino-phenoxy)-1*H*-pyrazol,
3-(2,4-Dihydroxy-phenyl)-4-(3-amino-phenoxy)-1*H*-pyrazol,
3-(2,4-Dihydroxy-phenyl)-4-(4-amino-phenoxy)-1*H*-pyrazol,
3-(2,4-Dihydroxy-phenyl)-4-(4-cyan-phenoxy)-1*H*-pyrazol,
3-(2,4-Dihydroxy-phenyl)-4-(2-cyan-phenoxy)-1*H*-pyrazol,
3-(2,4-Dihydroxy-phenyl)-4-(3-cyan-phenoxy)-1*H*-pyrazol,
3-(2,4-Dihydroxy-phenyl)-4-(2,4-dimethyl-phenoxy)-1*H*-pyrazol,
3-(2,4-Dihydroxy-phenyl)-4-(2,5-dimethyl-phenoxy)-1*H*-pyrazol,
3-(2,4-Dihydroxy-phenyl)-4-(2,6-dimethyl-phenoxy)-1*H*-pyrazol,
3-(2,4-Dihydroxy-phenyl)-4-(3,4-dimethyl-phenoxy)-1*H*-pyrazol,
3-(2,4-Dihydroxy-phenyl)-4-(2-ethyl-phenoxy)-1*H*-pyrazol,
3-(2,4-Dihydroxy-phenyl)-4-(3-ethyl-phenoxy)-1*H*-pyrazol,
3-(2,4-Dihydroxy-phenyl)-4-(3,5-dimethyl-phenoxy)-1*H*-pyrazol,
3-(2,4-Dihydroxy-phenyl)-4-(4-ethyl-phenoxy)-1*H*-pyrazol,
3-(2,4-Dihydroxy-phenyl)-4-(2,3-dimethyl-phenoxy)-1*H*-pyrazol,
3-(2,4-Dihydroxy-phenyl)-4-(4-amino-3-methyl-phenoxy)-1*H-*pyrazol,
3-(2,4-Dihydroxy-phenyl)-4-(2-amino-5-methyl-phenoxy)-1*H-*pyrazol,
3-(2,4-Dihydroxy-phenyl)-4-(2-amino-4-methyl-phenoxy)-1*H-*pyrazol,
3-(2,4-Dihydroxy-phenyl)-4-(3-amino-6-methyl-phenoxy)-1*H-*pyrazol,
3-(2,4-Dihydroxy-phenyl)-4-(4-amino-3-fluor-phenoxy)-1*H-*pyrazol,
3-(2,4-Dihydroxy-phenyl)-4-(3,4-difluor-phenoxy)-1*H*-pyrazol,
3-(2,4-Dihydroxy-phenyl)-4-(2,3-difluor-phenoxy)-1*H*-pyrazol,
3-(2,4-Dihydroxy-phenyl)-4-(3,5-difluor-phenoxy)-1*H*-pyrazol,
3-(2,4-Dihydroxy-phenyl)-4-(2,4-difluor-phenoxy)-1*H*-pyrazol,
3-(2,4-Dihydroxy-phenyl)-4-(2,6-difluor-phenoxy)-1*H*-pyrazol,
3-(2,4-Dihydroxy-phenyl)-4-(indol-6-yloxy)-1*H*-pyrazol,
3-(2,4-Dihydroxy-phenyl)-4-(indol-4-yloxy)-1*H*-pyrazol,
3-(2,4-Dihydroxy-phenyl)-4-(indol-5-yloxy)-1*H*-pyrazol,
3-(2,4-Dihydroxy-phenyl)-4-(4-cyan-3-fluor-phenoxy)-1*H*-pyrazol,
3-(2,4-Dihydroxy-phenyl)-4-(2-aminocarbonyl-phenoxy)-1*H-*pyrazol,
3-(2,4-Dihydroxy-phenyl)-4-(4-aminocarbonyl-phenoxy)-1*H-*pyrazol,
3-(2,4-Dihydroxy-phenyl)-4-(4-acetamido-phenoxy)-1*H*-pyrazol,
3-(2,4-Dihydroxy-phenyl)-4-(2-acetamido-phenoxy)-1*H*-pyrazol,
3-(2,4-Dihydroxy-phenyl)-4-(3-acetamido-phenoxy)-1*H*-pyrazol,
3-(2,4-Dihydroxy-phenyl)-4-(3-ureido-phenoxy)-1*H*-pyrazol,
3-(2,4-Dihydroxy-phenyl)-4-(3-dimethylamino-phenoxy)-1*H-*pyrazol,
3-(2,4-Dihydroxy-phenyl)-4-(3-methoxycarbonyl-phenoxy)-1*H-*pyrazol,
3-(2,4-Dihydroxy-phenyl)-4-(2-methoxycarbonyl-phenoxy)-1*H-*pyrazol,
3-(2,4-Dihydroxy-phenyl)-4-[(4-fluor-phenyl)-sulfanyl]-1*H-*pyrazol,
3-(2,4-Dihydroxy-5-brom-phenyl)-4-(3-methyl-phenoxy)-1*H-*pyrazol,
3-(2,4-Dihydroxy-5-brom-phenyl)-4-(2,3-dibrom-phenoxy)-1*H-*pyrazol,
3-(2,4-Dihydroxy-5-brom-phenyl)-4-(4-fluor-phenoxy)-1*H-*pyrazol,
3-(2,4-Dihydroxy-5-brom-phenyl)-4-(3-chlor-phenoxy)-1*H-*pyrazol,
3-(2,4-Dihydroxy-5-brom-phenyl)-4-(4-chlor-phenoxy)-1*H-*pyrazol,
3-(2,4-Dihydroxy-5-brom-phenyl)-4-(4-ethyl-phenoxy)-1*H-*pyrazol,
3-(2,4-Dihydroxy-5-brom-phenyl)-4-(2,5-dimethyl-phenoxy)-1*H-*pyrazol,
3-(2,4-Dihydroxy-5-brom-phenyl)-4-(4-cyan-phenoxy)-1*H-*pyrazol,
3-(2,4-Dimethoxy-phenyl)-4-[(4-fluor-phenyl)-sulfanyl]-1*H-*pyrazol,
3-(2,4-Dihydroxy-5-brom-phenyl)-4-(3-dimethylamino-phenoxy)-1*H*-pyrazol,
3-(2,4-Dihydroxy-5-brom-phenyl)-4-(2,6-difluor-phenoxy)-1*H-*pyrazol,
3-(2,4-Dihydroxy-5-brom-phenyl)-4-(2,4-difluor-phenoxy)-1*H-*pyrazol,
3-(2,4-Dihydroxy-5-brom-phenyl)-4-(2-chlor-phenoxy)-1*H-*pyrazol,
3-(2,4-Dihydroxy-5-brom-phenyl)-4-(2,3-dimethyl-phenoxy)-1*H-*pyrazol,
3-(2,4-Dihydroxy-5-brom-phenyl)-4-(3,5-dimethyl-phenoxy)-1*H-*pyrazol,
3-(2,4-Dihydroxy-5-brom-phenyl)-4-(3-ethyl-phenoxy)-1*H-*pyrazol,
3-(2,4-Dihydroxy-5-brom-phenyl)-4-(2-ethyl-phenoxy)-1*H-*pyrazol,
3-(2,4-Dihydroxy-5-brom-phenyl)-4-(3,4-dimethyl-phenoxy)-1*H-*pyrazol,
3-(2,4-Dihydroxy-5-brom-phenyl)-4-(2,4-dimethyl-phenoxy)-1*H-*pyrazol,
3-(2,4-Dihydroxy-5-brom-phenyl)-4-(2-fluor-phenoxy)-1*H-*pyrazol,
3-(2,4-Dihydroxy-5-brom-phenyl)-4-(2-methyl-phenoxy)-1*H-*pyrazol,
3-[2,4-Dihydroxy-5-(3-hydroxymethyl-piperidin-1-sulfonyl)-phenyl]-4-(2-chlor-phenoxy)-1*H*-pyrazol,
3-[2,4-Dihydroxy-5-diethylsulfamoyl-phenyl]-4-(2-chlor-4-diethylsulfamoyl-phenoxy)-1*H*-pyrazol,
3-[2,4-Dihydroxy-5-(pyridin-4-yl-sulfamoyl)-phenyl]-4-(2-chlor-phenoxy)-1*H*-pyrazol,
3-[2,4-Dihydroxy-5-(pyridin-2-yl-sulfamoyl)-phenyl]-4-(2-chlor-phenoxy)-1*H*-pyrazol,
3-[2,4-Dihydroxy-5-(pyridin-3-yl-sulfamoyl)-phenyl]-4-(2-chlor-phenoxy)-1*H*-pyrazol,
3-[2,4-Dihydroxy-5-(benzyl-sulfamoyl)-phenyl]-4-(2-chlor-phenoxy)-1*H*-pyrazol,
3-[2,4-Dihydroxy-5-(4-fluorphenyl-sulfamoyl)-phenyl]-4-(2-chlor-phenoxy)-1*H*-pyrazol,
3-[2,4-Dihydroxy-5-(3-fluorphenyl-sulfamoyl)-phenyl]-4-(2-chlor-phenoxy)-1*H*-pyrazol,
3-[2,4-Dihydroxy-5-(2-fluorphenyl-sulfamoyl)-phenyl]-4-(2-chlor-phenoxy)-1*H*-pyrazol,
3-[2,4-Dihydroxy-5-(phenyl-sulfamoyl)-phenyl]-4-(2-chlor-phenoxy)-1*H*-pyrazol,
3-[2,4-Dihydroxy-5-(2-diethylaminomethyl-piperidin-1-sulfonyl)-phenyl]-4-(2-chlor-phenoxy)-1*H*-pyrazol,
3-[2,4-Dihydroxy-5-(4-methyl-piperazin-1-sulfonyl)-phenyl]-4-(2-chlor-phenoxy)-1 H-pyrazol,
3-[2,4-Dihydroxy-5-(piperidin-1-sulfonyl)-phenyl]-4-(2-chlor-phenoxy)-1*H*-pyrazol,
3-[2,4-Dihydroxy-5-(diethyl-sulfamoyl)-phenyl]-4-(2-chlor-phenoxy)-1*H*-pyrazol,
sowie ihre pharmazeutisch verwendbaren Salze, Solvate, Tautomere und Stereoisomere, einschließlich deren Mischungen in allen Verhältnissen.

2. Arzneimittel enthaltend mindestens eine Verbindung gemäß Anspruch 1, ausgewählt aus der Gruppe
3-(2,4-Dihydroxy-phenyl)-4-(4-fluor-phenoxy)-1*H*-pyrazol,
3-(2,4-Dihydroxy-5-ethyl-phenyl)-4-(4-fluor-phenoxy)-5-methyl-1 H-pyrazol,
3-(2,4-Dihydroxy-phenyl)-4-(4-fluor-phenoxy)-5-trifluormethyl-1*H*-pyrazol,
3-(2,4-Dihydroxy-phenyl)-4-(4-ethoxy-phenoxy)-1*H*-pyrazol,
3-(2,4-Dihydroxy-phenyl)-4-phenoxy-1 H-pyrazol,
3-(2,4-Dihydroxy-phenyl)-4-(4-methyl-phenoxy)-1*H*-pyrazol,
3-(2,4-Dihydroxy-phenyl)-4-(3-methyl-phenoxy)-1*H*-pyrazol,
3-(2,4-Dihydroxy-phenyl)-4-(2-methyl-phenoxy)-1*H*-pyrazol,
3-(2,4-Dihydroxy-phenyl)-4-(2-amino-phenoxy)-1*H*-pyrazol,
3-(2,4-Dihydroxy-phenyl)-4-(3-amino-phenoxy)-1*H*-pyrazol,
3-(2,4-Dihydroxy-phenyl)-4-(4-amino-phenoxy)-1*H*-pyrazol,
3-(2,4-Dihydroxy-phenyl)-4-(3-fluor-phenoxy)-1*H*-pyrazol,
3-(2,4-Dihydroxy-phenyl)-4-(2-fluor-phenoxy)-1*H*-pyrazol,
3-(2,4-Dihydroxy-phenyl)-4-(4-cyan-phenoxy)-1*H*-pyrazol,
3-(2,4-Dihydroxy-phenyl)-4-(2-cyan-phenoxy)-1*H*-pyrazol,
3-(2,4-Dihydroxy-phenyl)-4-(3-cyan-phenoxy)-1*H*-pyrazol,
3-(2,4-Dihydroxy-phenyl)-4-(2,4-dimethyl-phenoxy)-1*H*-pyrazol,
3-(2,4-Dihydroxy-phenyl)-4-(2,5-dimethyl-phenoxy)-1*H*-pyrazol,
3-(2,4-Dihydroxy-phenyl)-4-(2,6-dimethyl-phenoxy)-1*H*-pyrazol,
3-(2,4-Dihydroxy-phenyl)-4-(3,4-dimethyl-phenoxy)-1*H*-pyrazol,
3-(2,4-Dihydroxy-phenyl)-4-(2-ethyl-phenoxy)-1*H*-pyrazol,
3-(2,4-Dihydroxy-phenyl)-4-(3-ethyl-phenoxy)-1*H*-pyrazol,
3-(2,4-Dihydroxy-phenyl)-4-(3,5-dimethyl-phenoxy)-1*H*-pyrazol,
3-(2,4-Dihydroxy-phenyl)-4-(4-ethyl-phenoxy)-1*H*-pyrazol,
3-(2,4-Dihydroxy-phenyl)-4-(2,3-dimethyl-phenoxy)-1*H*-pyrazol,
3-(2,4-Dihydroxy-phenyl)-4-(4-amino-3-methyl-phenoxy)-1*H-*pyrazol,
3-(2,4-Dihydroxy-phenyl)-4-(2-amino-5-methyl-phenoxy)-1*H-*pyrazol,
3-(2,4-Dihydroxy-phenyl)-4-(2-amino-4-methyl-phenoxy)-1*H-*pyrazol,
3-(2,4-Dihydroxy-phenyl)-4-(3-amino-6-methyl-phenoxy)-1*H-*pyrazol,
3-(2,4-Dihydroxy-phenyl)-4-(4-amino-3-fluor-phenoxy)-1*H-*pyrazol,
3-(2,4-Dihydroxy-phenyl)-4-(2-chlor-phenoxy)-1*H*-pyrazol,
3-(2,4-Dihydroxy-phenyl)-4-(4-chlor-phenoxy)-1*H*-pyrazol,
3-(2,4-Dihydroxy-phenyl)-4-(3-chlor-phenoxy)-1*H*-pyrazol,
3-(2,4-Dihydroxy-phenyl)-4-(3,4-difluor-phenoxy)-1*H*-pyrazol,
3-(2,4-Dihydroxy-phenyl)-4-(2,3-difluor-phenoxy)-1*H*-pyrazol,
3-(2,4-Dihydroxy-phenyl)-4-(3,5-difluor-phenoxy)-1*H*-pyrazol,
3-(2,4-Dihydroxy-phenyl)-4-(2,4-difluor-phenoxy)-1*H*-pyrazol,
3-(2,4-Dihydroxy-phenyl)-4-(2,6-difluor-phenoxy)-1*H*-pyrazol,
3-(2,4-Dihydroxy-phenyl)-4-(indol-6-yloxy)-1*H*-pyrazol,
3-(2,4-Dihydroxy-phenyl)-4-(indol-4-yloxy)-1*H*-pyrazol,
3-(2,4-Dihydroxy-phenyl)-4-(indol-5-yloxy)-1*H*-pyrazol,
3-(2,4-Dihydroxy-phenyl)-4-(4-cyan-3-fluor-phenoxy)-1*H*-pyrazol,
3-(2,4-Dihydroxy-phenyl)-4-(2-aminocarbonyl-phenoxy)-1*H-*pyrazol,
3-(2,4-Dihydroxy-phenyl)-4-(4-aminocarbonyl-phenoxy)-1*H-*pyrazol,
3-(2,4-Dihydroxy-phenyl)-4-(4-acetamido-phenoxy)-1*H*-pyrazol,
3-(2,4-Dihydroxy-phenyl)-4-(2-acetamido-phenoxy)-1*H*-pyrazol,
3-(2,4-Dihydroxy-phenyl)-4-(3-acetamido-phenoxy)-1*H*-pyrazol,
3-(2,4-Dihydroxy-phenyl)-4-(3-ureido-phenoxy)-1*H*-pyrazol,
3-(2,4-Dihydroxy-phenyl)-4-(3-dimethylamino-phenoxy)-1*H-*pyrazol,
3-(2,4-Dihydroxy-phenyl)-4-(3-methoxycarbonyl-phenoxy)-1*H-*pyrazol,
3-(2,4-Dihydroxy-phenyl)-4-(2-methoxycarbonyl-phenoxy)-1*H-*pyrazol,
3-(2,4-Dihydroxy-phenyl)-4-[(4-fluor-phenyl)-sulfanyl]-1*H-*pyrazol,
3-(2,4-Dihydroxy-5-brom-phenyl)-4-(3-methyl-phenoxy)-1*H-*pyrazol,
3-(2,4-Dihydroxy-5-brom-phenyl)-4-(2,3-dibrom-phenoxy)-1*H-*pyrazol,
3-(2,4-Dihydroxy-5-brom-phenyl)-4-(4-fluor-phenoxy)-1*H-*pyrazol,
3-(2,4-Dihydroxy-5-brom-phenyl)-4-(3-chlor-phenoxy)-1*H-*pyrazol,
3-(2,4-Dihydroxy-5-brom-phenyl)-4-(4-chlor-phenoxy)-1*H-*pyrazol,
3-(2,4-Dihydroxy-5-brom-phenyl)-4-(4-ethyl-phenoxy)-1*H-*pyrazol,
3-(2,4-Dihydroxy-5-brom-phenyl)-4-(2,5-dimethyl-phenoxy)-1*H-*pyrazol,
3-(2,4-Dihydroxy-5-brom-phenyl)-4-(4-cyan-phenoxy)-1*H-*pyrazol,
3-(2,4-Dimethoxy-phenyl)-4-[(4-fluor-phenyl)-sulfanyl]-1*H-*pyrazol,
3-(2,4-Dihydroxy-phenyl)-4-(3-carboxy-phenoxy)-1*H*-pyrazol,
3-(2,4-Dihydroxy-5-brom-phenyl)-4-(3-dimethylamino-phenoxy)-1*H*-pyrazol,
3-(2,4-Dihydroxy-5-brom-phenyl)-4-(2,6-difluor-phenoxy)-1*H-*pyrazol,
3-(2,4-Dihydroxy-5-brom-phenyl)-4-(2,4-difluor-phenoxy)-1*H-*pyrazol,
3-(2,4-Dihydroxy-5-brom-phenyl)-4-(2-chlor-phenoxy)-1*H-*pyrazol,
3-(2,4-Dihydroxy-5-brom-phenyl)-4-(2,3-dimethyl-phenoxy)-1*H-*pyrazol,
3-(2,4-Dihydroxy-5-brom-phenyl)-4-(3,5-dimethyl-phenoxy)-1*H-*pyrazol,
3-(2,4-Dihydroxy-5-brom-phenyl)-4-(3-ethyl-phenoxy)-1*H-*pyrazol,
3-(2,4-Dihydroxy-5-brom-phenyl)-4-(2-ethyl-phenoxy)-1*H-*pyrazol,
3-(2,4-Dihydroxy-5-brom-phenyl)-4-(3,4-dimethyl-phenoxy)-1*H-*pyrazol,
3-(2,4-Dihydroxy-5-brom-phenyl)-4-(2,4-dimethyl-phenoxy)-1*H-*pyrazol,
3-(2,4-Dihydroxy-5-brom-phenyl)-4-(2-fluor-phenoxy)-1*H-*pyrazol,
3-(2,4-Dihydroxy-5-brom-phenyl)-4-(2-methyl-phenoxy)-1*H-*pyrazol,
3-[2,4-Dihydroxy-5-(3-hydroxymethyl-piperidin-1-sulfonyl)-phenyl]-4-(2-chlor-phenoxy)-1*H*-pyrazol,
3-[2,4-Dihydroxy-5-diethylsulfamoyl-phenyl]-4-(2-chlor-4-diethylsulfamoyl-phenoxy)-1*H*-pyrazol,
3-[2,4-Dihydroxy-5-(pyridin-4-yl-sulfamoyl)-phenyl]-4-(2-chlor-phenoxy)-1*H*-pyrazol,
3-[2,4-Dihydroxy-5-(pyridin-2-yl-sulfamoyl)-phenyl]-4-(2-chlor-phenoxy)-1*H*-pyrazol,
3-[2,4-Dihydroxy-5-(pyridin-3-yl-sulfamoyl)-phenyl]-4-(2-chlor-phenoxy)-1*H*-pyrazol,
3-[2,4-Dihydroxy-5-(benzyl-sulfamoyl)-phenyl]-4-(2-chlor-phenoxy)-1*H*-pyrazol,
3-[2,4-Dihydroxy-5-(4-fluorphenyl-sulfamoyl)-phenyl]-4-(2-chlor-phenoxy)-1*H*-pyrazol,
3-[2,4-Dihydroxy-5-(3-fluorphenyl-sulfamoyl)-phenyl]-4-(2-chlor-phenoxy)-1*H*-pyrazol,
3-[2,4-Dihydroxy-5-(2-fluorphenyl-sulfamoyl)-phenyl]-4-(2-chlor-phenoxy)-1*H*-pyrazol,
3-[2,4-Dihydroxy-5-(phenyl-sulfamoyl)-phenyl]-4-(2-chlor-phenoxy)-1*H*-pyrazol,
3-[2,4-Dihydroxy-5-(2-diethylaminomethyl-piperidin-1-sulfonyl)-phenyl]-4-(2-chlor-phenoxy)-1*H*-pyrazol,
3-[2,4-Dihydroxy-5-(4-methyl-piperazin-1-sulfonyl)-phenyl]-4-(2-chlor-phenoxy)-1*H*-pyrazol,
3-[2,4-Dihydroxy-5-(piperidin-1-sulfonyl)-phenyl]-4-(2-chlor-phenoxy)-1*H*-pyrazol,
3-[2,4-Dihydroxy-5-(diethyl-sulfamoyl)-phenyl]-4-(2-chlor-phenoxy)-1*H*-pyrazol,
sowie ihre pharmazeutisch verwendbaren Salze, Solvate, Tautomere und Stereoisomere, einschließlich deren Mischungen in allen Verhältnissen.

3. Verwendung von Verbindungen gemäß Anspruch 1, sowie ihrer pharmazeutisch verwendbaren Salze, Solvate, Tautomere und Stereoisomere, einschließlich deren Mischungen in allen Verhältnissen, zur Herstellung eines Arzneimittels zur Behandlung und/oder Prophylaxe von Krankheiten, bei denen die Hemmung, Regulierung und/oder Modulation von HSP90 eine Rolle spielt.

4. Verwendung nach Anspruch 3 von Verbindungen gemäß Anspruch 1, sowie ihrer pharmazeutisch verwendbaren Salze, Solvate, Tautomere und Stereoisomere, einschließlich deren Mischungen in allen Verhältnissen, zur Herstellung eines Arzneimittels zur Behandlung oder Vorbeugung von Tumorerkrankungen, viralen Erkrankungen, zur Immunsuppression bei Transplantationen, entzündungsbedingten Erkrankungen, Zystische Fibrose, Erkrankungen im Zusammenhang mit Angiogenese, infektiösen Erkrankungen, Autoimmunerkrankungen, Ischämie, fibrogenetischen Erkrankungen,
zur Förderung der Nervenregeneration,
zur Hemmung des Wachstums von Krebs, Tumorzellen und Tumormetastasen,
zum Schutz normaler Zellen gegen Toxizität, die durch Chemotherapie verursacht ist,
zur Behandlung von Krankheiten, wobei Proteinfehlfaltung oder Aggregation ein Hauptkausalfaktor ist.

5. Verwendung nach Anspruch 4, wobei es sich bei den Tumorerkrankungen um Fibrosarkom, Myxosarkom, Liposarkom, Chondrosarkom, osteogenem Sarkom, Chordom, Angiosarkom, Endotheliosarkom, Lymphangiosarkom, Lymphangioendotheliosarkom, Synoviom, Mesotheliom, Ewing-Tumor, Leiosarkom, Rhabdomyosarkom, Kolonkarzinom, Pankreaskrebs, Brustkrebs, Ovarkrebs, Prostatakrebs, Plattenzellkarzinom, Basalzellkarzinom, Adenokarzinom, Schweißdrüsenkarzinom, Talgdrüsenkarzinom, Papillarkarzinom, Papillaradenokarzinomen, Cystadenokarzinomen, Knochenmarkkarzinom, bronchogenem Karzinom, Nierenzellkarzinom, Hepatom, Gallengangkarzinom, Chorionkarzinom, Seminom, embryonalem Karzinom, Wilms-Tumor, Cervix-Krebs, Hodentumor, Lungenkarzinom, kleinzelligem Lungenkarzinom, Blasenkarzinom, Epithelkarzinom, Gliom, Astrocytom, Medulloblastom, Kraniopharyngiom, Ependymom, Pinealom, Hämangioblastom, akustischem Neurom, Oligodendrogliom, Meningiom, Melanom, Neuroblastom, Retinoblastom, Leukämie, Lymphom, multiplem Myelom, Waldenströms Makroglobulinämie und Schwere-Kettenerkrankung handelt.

6. Verwendung nach Anspruch 4, wobei das virale Pathogen der viralen Erkrankungen ausgewählt ist aus der Gruppe, bestehend aus Hepatitis Typ A, Hepatitis Typ B, Hepatitis Typ C, Influenza, Varicella, Adenovirus, Herpes-Simplex Typ I (HSV-I), Herpes Simplex Typ II (HSV-II), Rinderpest, Rhinovirus, Echovirus, Rotavirus, respiratorischem Synzytialvirus (RSV), Papillomvirus, Papovavirus, Cytomegalievirus, Echinovirus, Arbovirus, Huntavirus, Coxsackievirus, Mumpsvirus, Masernvirus, Rötelnvirus, Poliovirus, menschliches Immunschwächevirus Typ I (HIV-I) und menschliches Immunschwächevirus Typ II (HIV-II).

7. Verwendung nach Anspruch 4, wobei es sich bei den entzündungsbedingten Erkrankungen um Rheumatoide Arthritis, Asthma, Multiple Sklerose, Typ 1 Diabetes, Lupus Erythematodes, Psoriasis und Inflammatory Bowel Disease handelt.

8. Verwendung nach Anspruch 4, wobei es sich bei den Erkrankungen im Zusammenhang mit Angiogenese um diabetische Retinopathie, Hämangiome, Endometriose und Tumorangiogenese handelt.

9. Verwendung nach Anspruch 4, wobei es sich bei den fibrogenetischen Erkrankungen um Sklerodermie, Polymyositis, systemischer Lupus, Leberzirrhose, Keloidbildung, interstitielle Nephritis und pulmonare Fibrose handelt.

10. Verwendung nach Anspruch 4, wobei es sich bei den Krankheiten, bei denen Proteinfehlfaltung oder Aggregation ein Hauptkausalfaktor ist, um Skrapie, Creutzfeldt-Jakob-Krankheit, Huntington oder Alzheimer handelt.

11. Arzneimittel enthaltend mindestens eine Verbindung gemäß Anspruch 1 und/oder ihre pharmazeutisch verwendbaren Salze, Solvate, Tautomere und Stereoisomere, einschließlich deren Mischungen in allen Verhältnissen, und mindestens einen weiteren Arzneimittelwirkstoff.

12. Set (Kit), bestehend aus getrennten Packungen von
(a) einer wirksamen Menge an einer Verbindung gemäß Anspruch 1 und/oder ihrer pharmazeutisch verwendbaren Salze, Solvate, Tautomere und Stereoisomere, einschließlich deren Mischungen in allen Verhältnissen,
und
(b) einer wirksamen Menge eines weiteren Arzneimittelswirkstoffs.

## Claims

1. Compounds selected from the group
3-(2,4-dihydroxyphenyl)-4-(4-methylphenoxy)-1*H*-pyrazole,
3-(2,4-dihydroxyphenyl)-4-(3-methylphenoxy)-1*H*-pyrazole,
3-(2,4-dihydroxyphenyl)-4-(2-methylphenoxy)-1*H*-pyrazole,
3-(2,4-dihydroxyphenyl)-4-(2-aminophenoxy)-1*H*-pyrazole,
3-(2,4-dihydroxyphenyl)-4-(3-aminophenoxy)-1*H*-pyrazole,
3-(2,4-dihydroxyphenyl)-4-(4-aminophenoxy)-1*H*-pyrazole,
3-(2,4-dihydroxyphenyl)-4-(4-cyanophenoxy)-1*H*-pyrazole,
3-(2,4-dihydroxyphenyl)-4-(2-cyanophenoxy)-1*H*-pyrazole,
3-(2,4-dihydroxyphenyl)-4-(3-cyanophenoxy)-1*H*-pyrazole,
3-(2,4-dihydroxyphenyl)-4-(2,4-dimethylphenoxy)-1*H*-pyrazole,
3-(2,4-dihydroxyphenyl)-4-(2,5-dimethylphenoxy)-1*H*-pyrazole,
3-(2,4-dihydroxyphenyl)-4-(2,6-dimethylphenoxy)-1*H*-pyrazole,
3-(2,4-dihydroxyphenyl)-4-(3,4-dimethylphenoxy)-1*H*-pyrazole,
3-(2,4-dihydroxyphenyl)-4-(2-ethylphenoxy)-1*H*-pyrazole,
3-(2,4-dihydroxyphenyl)-4-(3-ethylphenoxy)-1*H*-pyrazole,
3-(2,4-dihydroxyphenyl)-4-(3,5-dimethylphenoxy)-1*H*-pyrazole,
3-(2,4-dihydroxyphenyl)-4-(4-ethylphenoxy)-1*H*-pyrazole,
3-(2,4-dihydroxyphenyl)-4-(2,3-dimethylphenoxy)-1*H*-pyrazole,
3-(2,4-dihydroxyphenyl)-4-(4-amino-3-methylphenoxy)-1*H-*pyrazole,
3-(2,4-dihydroxyphenyl)-4-(2-amino-5-methylphenoxy)-1*H-*pyrazole,
3-(2,4-dihydroxyphenyl)-4-(2-amino-4-methylphenoxy)-1*H-*pyrazole,
3-(2,4-dihydroxyphenyl)-4-(3-amino-6-methylphenoxy)-1*H-*pyrazole,
3-(2,4-dihydroxyphenyl)-4-(4-amino-3-fluorophenoxy)-1*H-*pyrazole,
3-(2,4-dihydroxyphenyl)-4-(3,4-difluorophenoxy)-1*H*-pyrazole,
3-(2,4-dihydroxyphenyl)-4-(2,3-difluorophenoxy)-1*H*-pyrazole,
3-(2,4-dihydroxyphenyl)-4-(3,5-difluorophenoxy)-1*H*-pyrazole,
3-(2,4-dihydroxyphenyl)-4-(2,4-difluorophenoxy)-1*H*-pyrazole,
3-(2,4-dihydroxyphenyl)-4-(2,6-difluorophenoxy)-1*H*-pyrazole,
3-(2,4-dihydroxyphenyl)-4-(indol-6-yloxy)-1*H*-pyrazole,
3-(2,4-dihydroxyphenyl)-4-(indol-4-yloxy)-1*H*-pyrazole,
3-(2,4-dihydroxyphenyl)-4-(indol-5-yloxy)-1*H*-pyrazole,
3-(2,4-dihydroxyphenyl)-4-(4-cyano-3-fluorophenoxy)-1*H-*pyrazole,
3-(2,4-dihydroxyphenyl)-4-(2-aminocarbonylphenoxy)-1*H-*pyrazole,
3-(2,4-dihydroxyphenyl)-4-(4-aminocarbonylphenoxy)-1*H-*pyrazole,
3-(2,4-dihydroxyphenyl)-4-(4-acetamidophenoxy)-1*H*-pyrazole,
3-(2,4-dihydroxyphenyl)-4-(2-acetamidophenoxy)-1*H*-pyrazole,
3-(2,4-dihydroxyphenyl)-4-(3-acetamidophenoxy)-1*H*-pyrazole,
3-(2,4-dihydroxyphenyl)-4-(3-ureidophenoxy)-1*H*-pyrazole,
3-(2,4-dihydroxyphenyl)-4-(3-dimethylaminophenoxy)-1*H-*pyrazole,
3-(2,4-dihydroxyphenyl)-4-(3-methoxycarbonylphenoxy)-1*H-*pyrazole,
3-(2,4-dihydroxyphenyl)-4-(2-methoxycarbonylphenoxy)-1*H-*pyrazole,
3-(2,4-dihydroxyphenyl)-4-[(4-fluorophenyl)sulfanyl]-1*H-*pyrazole,
3-(2,4-dihydroxy-5-bromophenyl)-4-(3-methylphenoxy)-1*H-*pyrazole,
3-(2,4-dihydroxy-5-bromophenyl)-4-(2,3-dibromophenoxy)-1*H-*pyrazole,
3-(2,4-dihydroxy-5-bromophenyl)-4-(4-fluorophenoxy)-1*H-*pyrazole,
3-(2,4-dihydroxy-5-bromophenyl)-4-(3-chlorophenoxy)-1*H-*pyrazole,
3-(2,4-dihydroxy-5-bromophenyl)-4-(4-chlorophenoxy)-1*H-*pyrazole,
3-(2,4-dihydroxy-5-bromophenyl)-4-(4-ethylphenoxy)-1*H-*pyrazole,
3-(2,4-dihydroxy-5-bromophenyl)-4-(2,5-dimethylphenoxy)-1*H-*pyrazole,
3-(2,4-dihydroxy-5-bromophenyl)-4-(4-cyanophenoxy)-1*H-*pyrazole,
3-(2,4-dimethoxyphenyl)-4-[(4-fluorophenyl)sulfanyl]-1*H-*pyrazole,
3-(2,4-dihydroxy-5-bromophenyl)-4-(3-dimethylaminophenoxy)-1*H*-pyrazole,
3-(2,4-dihydroxy-5-bromophenyl)-4-(2,6-difluorophenoxy)-1*H-*pyrazole,
3-(2,4-dihydroxy-5-bromophenyl)-4-(2,4-difluorophenoxy)-1*H-*pyrazole,
3-(2,4-dihydroxy-5-bromophenyl)-4-(2-chlorophenoxy)-1*H-*pyrazole,
3-(2,4-dihydroxy-5-bromophenyl)-4-(2,3-dimethylphenoxy)-1*H-*pyrazole,
3-(2,4-dihydroxy-5-bromophenyl)-4-(3,5-dimethylphenoxy)-1*H-*pyrazole,
3-(2,4-dihydroxy-5-bromophenyl)-4-(3-ethylphenoxy)-1*H-*pyrazole,
3-(2,4-dihydroxy-5-bromophenyl)-4-(2-ethylphenoxy)-1*H-*pyrazole,
3-(2,4-dihydroxy-5-bromophenyl)-4-(3,4-dimethylphenoxy)-1*H-*pyrazole,
3-(2,4-dihydroxy-5-bromophenyl)-4-(2,4-dimethylphenoxy)-1*H-*pyrazole,
3-(2,4-dihydroxy-5-bromophenyl)-4-(2-fluorophenoxy)-1*H-*pyrazole,
3-(2,4-dihydroxy-5-bromophenyl)-4-(2-methylphenoxy)-1*H-*pyrazole,
3-[2,4-dihydroxy-5-(3-hydroxymethylpiperidine-1-sulfonyl)-phenyl]-4-(2-chlorophenoxy)-1*H*-pyrazole,
3-[2,4-dihydroxy-5-diethylsulfamoylphenyl]-4-(2-chloro-4-di-ethylsulfamoylphenoxy)-1*H*-pyrazole,
3-[2,4-dihydroxy-5-(pyridin-4-ylsulfamoyl)phenyl]-4-(2-chloro-phenoxy)-1*H*-pyrazole,
3-[2,4-dihydroxy-5-(pyridin-2-ylsulfamoyl)phenyl]-4-(2-chloro-phenoxy)-1*H*-pyrazole,
3-[2,4-dihydroxy-5-(pyridin-3-ylsulfamoyl)phenyl]-4-(2-chloro-phenoxy)-1*H*-pyrazole,
3-[2,4-dihydroxy-5-(benzylsulfamoyl)phenyl]-4-(2-chloro-phenoxy)-1*H*-pyrazole,
3-[2,4-dihydroxy-5-(4-fluorophenylsulfamoyl)phenyl]-4-(2-chloro-phenoxy)-1*H*-pyrazole,
3-[2,4-dihydroxy-5-(3-fluorophenylsulfamoyl)phenyl]-4-(2-chloro-phenoxy)-1*H*-pyrazole,
3-[2,4-dihydroxy-5-(2-fluorophenylsulfamoyl)phenyl]-4-(2-chloro-phenoxy)-1*H*-pyrazole,
3-[2,4-dihydroxy-5-(phenylsulfamoyl)phenyl]-4-(2-chloro-phenoxy)-1*H*-pyrazole,
3-[2,4-dihydroxy-5-(2-diethylaminomethylpiperidine-1-sulfonyl)-phenyl]-4-(2-chlorophenoxy)-1*H*-pyrazole,
3-[2,4-dihydroxy-5-(4-methylpiperazine-1-sulfonyl)phenyl]-4-(2-chlorophenoxy)-1*H*-pyrazole,
3-[2,4-dihydroxy-5-(piperidine-1-sulfonyl)phenyl]-4-(2-chloro-phenoxy)-1*H*-pyrazole,
3-[2,4-dihydroxy-5-(diethylsulfamoyl)phenyl]-4-(2-chloro-phenoxy)-1*H*-pyrazole,
and pharmaceutically usable salts, solvates, tautomers and stereoisomers thereof, including mixtures thereof in all ratios.

2. Medicaments comprising at least one compound according to Claim 1, selected from the group
3-(2,4-dihydroxyphenyl)-4-(4-fluorophenoxy)-1*H*-pyrazole,
3-(2,4-dihydroxy-5-ethylphenyl)-4-(4-fluorophenoxy)-5-methyl-1*H*-pyrazole,
3-(2,4-dihydroxyphenyl)-4-(4-fluorophenoxy)-5-trifluoromethyl-1*H*-pyrazole,
3-(2,4-dihydroxyphenyl)-4-(4-ethoxyphenoxy)-1*H*-pyrazole,
3-(2,4-dihydroxyphenyl)-4-phenoxy-1*H*-pyrazole,
3-(2,4-dihydroxyphenyl)-4-(4-methylphenoxy)-1*H*-pyrazole,
3-(2,4-dihydroxyphenyl)-4-(3-methylphenoxy)-1*H*-pyrazole,
3-(2,4-dihydroxyphenyl)-4-(2-methylphenoxy)-1*H*-pyrazole,
3-(2,4-dihydroxyphenyl)-4-(2-aminophenoxy)-1*H*-pyrazole,
3-(2,4-dihydroxyphenyl)-4-(3-aminophenoxy)-1*H*-pyrazole,
3-(2,4-dihydroxyphenyl)-4-(4-aminophenoxy)-1*H*-pyrazole,
3-(2,4-dihydroxyphenyl)-4-(3-fluorophenoxy)-1*H*-pyrazole,
3-(2,4-dihydroxyphenyl)-4-(2-fluorophenoxy)-1*H*-pyrazole,
3-(2,4-dihydroxyphenyl)-4-(4-cyanophenoxy)-1*H*-pyrazole,
3-(2,4-dihydroxyphenyl)-4-(2-cyanophenoxy)-1*H*-pyrazole,
3-(2,4-dihydroxyphenyl)-4-(3-cyanophenoxy)-1*H*-pyrazole,
3-(2,4-dihydroxyphenyl)-4-(2,4-dimethylphenoxy)-1*H*-pyrazole,
3-(2,4-dihydroxyphenyl)-4-(2,5-dimethylphenoxy)-1*H*-pyrazole,
3-(2,4-dihydroxyphenyl)-4-(2,6-dimethylphenoxy)-1*H*-pyrazole,
3-(2,4-dihydroxyphenyl)-4-(3,4-dimethylphenoxy)-1*H*-pyrazole,
3-(2,4-dihydroxyphenyl)-4-(2-ethylphenoxy)-1*H*-pyrazole,
3-(2,4-dihydroxyphenyl)-4-(3-ethylphenoxy)-1*H*-pyrazole,
3-(2,4-dihydroxyphenyl)-4-(3,5-dimethylphenoxy)-1*H*-pyrazole,
3-(2,4-dihydroxyphenyl)-4-(4-ethylphenoxy)-1*H*-pyrazole,
3-(2,4-dihydroxyphenyl)-4-(2,3-dimethylphenoxy)-1*H*-pyrazole,
3-(2,4-dihydroxyphenyl)-4-(4-amino-3-methylphenoxy)-1*H-*pyrazole,
3-(2,4-dihydroxyphenyl)-4-(2-amino-5-methylphenoxy)-1*H-*pyrazole,
3-(2,4-dihydroxyphenyl)-4-(2-amino-4-methylphenoxy)-1*H-*pyrazole,
3-(2,4-dihydroxyphenyl)-4-(3-amino-6-methylphenoxy)-1*H-*pyrazole,
3-(2,4-dihydroxyphenyl)-4-(4-amino-3-fluorophenoxy)-1*H-*pyrazole,
3-(2,4-dihydroxyphenyl)-4-(2-chlorophenoxy)-1*H*-pyrazole,
3-(2,4-dihydroxyphenyl)-4-(4-chlorophenoxy)-1*H*-pyrazole,
3-(2,4-dihydroxyphenyl)-4-(3-chlorophenoxy)-1*H*-pyrazole,
3-(2,4-dihydroxyphenyl)-4-(3,4-difluorophenoxy)-1*H*-pyrazole,
3-(2,4-dihydroxyphenyl)-4-(2,3-difluorophenoxy)-1*H*-pyrazole,
3-(2,4-dihydroxyphenyl)-4-(3,5-difluorophenoxy)-1*H*-pyrazole,
3-(2,4-dihydroxyphenyl)-4-(2,4-difluorophenoxy)-1*H*-pyrazole,
3-(2,4-dihydroxyphenyl)-4-(2,6-difluorophenoxy)-1*H*-pyrazole,
3-(2,4-dihydroxyphenyl)-4-(indol-6-yloxy)-1*H*-pyrazole,
3-(2,4-dihydroxyphenyl)-4-(indol-4-yloxy)-1*H*-pyrazole,
3-(2,4-dihydroxyphenyl)-4-(indol-5-yloxy)-1*H*-pyrazole,
3-(2,4-dihydroxyphenyl)-4-(4-cyano-3-fluorophenoxy)-1*H-*pyrazole,
3-(2,4-dihydroxyphenyl)-4-(2-aminocarbonylphenoxy)-1*H-*pyrazole,
3-(2,4-dihydroxyphenyl)-4-(4-aminocarbonylphenoxy)-1*H-*pyrazole,
3-(2,4-dihydroxyphenyl)-4-(4-acetamidophenoxy)-1*H*-pyrazole,
3-(2,4-dihydroxyphenyl)-4-(2-acetamidophenoxy)-1*H*-pyrazole,
3-(2,4-dihydroxyphenyl)-4-(3-acetamidophenoxy)-1*H*-pyrazole,
3-(2,4-dihydroxyphenyl)-4-(3-ureidophenoxy)-1*H*-pyrazole,
3-(2,4-dihydroxyphenyl)-4-(3-dimethylaminophenoxy)-1*H-*pyrazole,
3-(2,4-dihydroxyphenyl)-4-(3-methoxycarbonylphenoxy)-1*H-*pyrazole,
3-(2,4-dihydroxyphenyl)-4-(2-methoxycarbonylphenoxy)-1*H-*pyrazole,
3-(2,4-dihydroxyphenyl)-4-[(4-fluorophenyl)sulfanyl]-1*H-*pyrazole,
3-(2,4-dihydroxy-5-bromophenyl)-4-(3-methylphenoxy)-1*H-*pyrazole,
3-(2,4-dihydroxy-5-bromophenyl)-4-(2,3-dibromophenoxy)-1*H-*pyrazole,
3-(2,4-dihydroxy-5-bromophenyl)-4-(4-fluorophenoxy)-1*H-*pyrazole,
3-(2,4-dihydroxy-5-bromophenyl)-4-(3-chlorophenoxy)-1*H-*pyrazole,
3-(2,4-dihydroxy-5-bromophenyl)-4-(4-chlorophenoxy)-1*H-*pyrazole,
3-(2,4-dihydroxy-5-bromophenyl)-4-(4-ethylphenoxy)-1*H-*pyrazole,
3-(2,4-dihydroxy-5-bromophenyl)-4-(2,5-dimethylphenoxy)-1*H-*pyrazole,
3-(2,4-dihydroxy-5-bromophenyl)-4-(4-cyanophenoxy)-1*H-*pyrazole,
3-(2,4-dimethoxyphenyl)-4-[(4-fluorophenyl)sulfanyl]-1*H-*pyrazole,
3-(2,4-dihydroxyphenyl)-4-(3-carboxyphenoxy)-1*H*-pyrazole,
3-(2,4-dihydroxy-5-bromophenyl)-4-(3-dimethylaminophenoxy)-1 H-pyrazole,
3-(2,4-dihydroxy-5-bromophenyl)-4-(2,6-difluorophenoxy)-1*H-*pyrazole,
3-(2,4-dihydroxy-5-bromophenyl)-4-(2,4-difluorophenoxy)-1*H-*pyrazole,
3-(2,4-dihydroxy-5-bromophenyl)-4-(2-chlorophenoxy)-1*H-*pyrazole,
3-(2,4-dihydroxy-5-bromophenyl)-4-(2,3-dimethylphenoxy)-1*H-*pyrazole,
3-(2,4-dihydroxy-5-bromophenyl)-4-(3,5-dimethylphenoxy)-1*H-*pyrazole,
3-(2,4-dihydroxy-5-bromophenyl)-4-(3-ethylphenoxy)-1*H-*pyrazole,
3-(2,4-dihydroxy-5-bromophenyl)-4-(2-ethylphenoxy)-1*H-*pyrazole,
3-(2,4-dihydroxy-5-bromophenyl)-4-(3,4-dimethylphenoxy)-1*H-*pyrazole,
3-(2,4-dihydroxy-5-bromophenyl)-4-(2,4-dimethylphenoxy)-1*H-*pyrazole,
3-(2,4-dihydroxy-5-bromophenyl)-4-(2-fluorophenoxy)-1*H-*pyrazole,
3-(2,4-dihydroxy-5-bromophenyl)-4-(2-methylphenoxy)-1*H-*pyrazole,
3-[2,4-dihydroxy-5-(3-hydroxymethylpiperidine-1-sulfonyl)-phenyl]-4-(2-chlorophenoxy)-1*H*-pyrazole,
3-[2,4-dihydroxy-5-diethylsulfamoylphenyl]-4-(2-chloro-4-di-ethylsulfamoylphenoxy)-1*H*-pyrazole,
3-[2,4-dihydroxy-5-(pyridin-4-ylsulfamoyl)phenyl]-4-(2-chloro-phenoxy)-1*H*-pyrazole,
3-[2,4-dihydroxy-5-(pyridin-2-ylsulfamoyl)phenyl]-4-(2-chloro-phenoxy)-1*H*-pyrazole,
3-[2,4-dihydroxy-5-(pyridin-3-ylsulfamoyl)phenyl]-4-(2-chloro-phenoxy)-1*H*-pyrazole,
3-[2,4-dihydroxy-5-(benzylsulfamoyl)phenyl]-4-(2-chloro-phenoxy)-1*H*-pyrazole,
3-[2,4-dihydroxy-5-(4-fluorophenylsulfamoyl)phenyl]-4-(2-chlorophenoxy)-1*H*-pyrazole,
3-[2,4-dihydroxy-5-(3-fluorophenylsulfamoyl)phenyl]-4-(2-chlorophenoxy)-1*H*-pyrazole,
3-[2,4-dihydroxy-5-(2-fluorophenylsulfamoyl)phenyl]-4-(2-chlorophenoxy)-1*H*-pyrazole,
3-[2,4-dihydroxy-5-(phenylsulfamoyl)phenyl]-4-(2-chloro-phenoxy)-1*H*-pyrazole,
3-[2,4-dihydroxy-5-(2-diethylaminomethylpiperidine-1-sulfonyl)-phenyl]-4-(2-chlorophenoxy)-1*H*-pyrazole,
3-[2,4-dihydroxy-5-(4-methylpiperazine-1-sulfonyl)phenyl]-4-(2-chlorophenoxy)-1*H*-pyrazole,
3-[2,4-dihydroxy-5-(piperidine-1-sulfonyl)phenyl]-4-(2-chloro-phenoxy)-1*H*-pyrazole,
3-[2,4-dihydroxy-5-(diethylsulfamoyl)phenyl]-4-(2-chloro-phenoxy)-1*H*-pyrazole,
and pharmaceutically usable salts, solvates, tautomers and stereoisomers thereof, including mixtures thereof in all ratios.

3. Use of compounds according to Claim 1, and pharmaceutically usable salts, solvates, tautomers and stereoisomers thereof, including mixtures thereof in all ratios, for the preparation of a medicament for the treatment and/or prophylaxis of diseases in which the inhibition, regulation and/or modulation of HSP90 plays a role.

4. Use according to Claim 3 of compounds according to Claim 1, and pharmaceutically usable salts, solvates, tautomers and stereoisomers thereof, including mixtures thereof in all ratios, for the preparation of a medicament for the treatment or prevention of tumour diseases, viral diseases, for immunosuppression in transplants, inflammation-induced diseases, cystic fibrosis, diseases associated with angiogenesis, infectious diseases, autoimmune diseases, ischaemia, fibrogenetic diseases,
for the promotion of nerve regeneration,
for inhibiting the growth of cancer, tumour cells and tumour metastases,
for the protection of normal cells against toxicity caused by chemotherapy,
for the treatment of diseases in which incorrect protein folding or aggregation is a principal causal factor.

5. Use according to Claim 4, where the tumour diseases are fibro-sarcoma, myxosarcoma, liposarcoma, chondrosarcoma, osteogenic sarcoma, chordoma, angiosarcoma, endotheliosarcoma, lymphangio-sarcoma, lymphangioendotheliosarcoma, synovioma, mesothelioma, Ewing's tumour, leiosarcoma, rhabdomyosarcoma, colon carcinoma, pancreatic cancer, breast cancer, ovarian cancer, prostate cancer, squamous cell carcinoma, basal cell carcinoma, adenocarcinoma, syringocarcinoma, sebaceous gland carcinoma, papillary carcinoma, papillary adenocarcinomas, cystadenocarcinomas, bone marrow carcinoma, bronchogenic carcinoma, renal cell carcinoma, hepatoma, bile duct carcinoma, choriocarcinoma, seminoma, embryonic carcinoma, Wilm's tumour, cervical cancer, testicular tumour, lung carcinoma, small-cell lung carcinoma, bladder carcinoma, epithelial carcinoma, glioma, astrocytoma, medulloblastoma, craniopharyngioma, ependymoma, pinealoma, haemangioblastoma, acoustic neuroma, oligodendroglioma, meningioma, melanoma, neuroblastoma, retino-blastoma, leukaemia, lymphoma, multiple myeloma, Waldenström's macroglobulinaemia and heavy-chain disease.

6. Use according to Claim 4, where the viral pathogen of the viral diseases is selected from the group consisting of hepatitis type A, hepatitis type B, hepatitis type C, influenza, varicella, adenovirus, herpes simplex type I (HSV-I), herpes simplex type II (HSV-II), cattle plague, rhinovirus, echovirus, rotavirus, respiratory syncytial virus (RSV), papillomavirus, papovavirus, cytomegalovirus, echinovirus, arbovirus, huntavirus, Coxsackie virus, mumps virus, measles virus, rubella virus, polio virus, human immunodeficiency virus type I (HIV-I) and human immunodeficiency virus type II (HIV-II).

7. Use according to Claim 4, where the inflammation-induced diseases are rheumatoid arthritis, asthma, multiple sclerosis, type 1 diabetes, lupus erythematosus, psoriasis and inflammatory bowel disease.

8. Use according to Claim 4, where the diseases associated with angiogenesis are diabetic retinopathy, haemangiomas, endometriosis and tumour angiogenesis.

9. Use according to Claim 4, where the fibrogenetic diseases are sclero-derma, polymyositis, systemic lupus, cirrhosis of the liver, keloid formation, interstitial nephritis and pulmonary fibrosis.

10. Use according to Claim 4, where the diseases in which incorrect protein folding or aggregation is a principal causal factor are scrapie, Creutzfeldt-Jakob disease, Huntington's or Alzheimer's.

11. Medicaments comprising at least one compound according to Claim 1 and/or pharmaceutically usable salts, solvates, tautomers and stereoisomers thereof, including mixtures thereof in all ratios, and at least one further medicament active compound.

12. Set (kit) consisting of separate packs of
(a) an effective amount of a compound according to Claim 1 and/or pharmaceutically usable salts, solvates, tautomers and stereoisomers thereof, including mixtures thereof in all ratios,
and
(b) an effective amount of a further medicament active compound.

## Revendications

1. Composés choisis dans le groupe constitué par
le 3-(2,4-dihydroxyphényl)-4-(4-méthylphénoxy)-1*H*-pyrazole,
le 3-(2,4-dihydroxyphényl)-4-(3-méthylphénoxy)-1*H*-pyrazole,
le 3-(2,4-dihydroxyphényl)-4-(2-méthylphénoxy)-1*H*-pyrazole,
le 3-(2,4-dihydroxyphényl)-4-(2-aminophénoxy)-1*H*-pyrazole,
le 3-(2,4-dihydroxyphényl)-4-(3-aminophénoxy)-1*H*-pyrazole,
le 3-(2,4-dihydroxyphényl)-4-(4-aminophénoxy)-1*H*-pyrazole,
le 3-(2,4-dihydroxyphényl)-4-(4-cyanophénoxy)-1*H*-pyrazole,
le 3-(2,4-dihydroxyphényl)-4-(2-cyanophénoxy)-1*H*-pyrazole,
le 3-(2,4-dihydroxyphényl)-4-(3-cyanophénoxy)-1*H*-pyrazole,
le 3-(2,4-dihydroxyphényl)-4-(2,4-diméthylphénoxy)-1*H-*pyrazole,
le 3-(2,4-dihydroxyphényl)-4-(2,5-diméthylphénoxy)-1*H-*pyrazole,
le 3-(2,4-dihydroxyphényl)-4-(2,6-diméthylphénoxy)-1*H-*pyrazole,
le 3-(2,4-dihydroxyphényl)-4-(3,4-diméthylphénoxy)-1*H-*pyrazole,
le 3-(2,4-dihydroxyphényl)-4-(2-éthylphénoxy)-1*H*-pyrazole,
le 3-(2,4-dihydroxyphényl)-4-(3-éthylphénoxy)-1*H*-pyrazole,
le 3-(2,4-dihydroxyphényl)-4-(3,5-diméthylphénoxy)-1*H-*pyrazole,
le 3-(2,4-dihydroxyphényl)-4-(4-éthylphénoxy)-1*H*-pyrazole,
le 3-(2,4-dihydroxyphényl)-4-(2,3-diméthylphénoxy)-1*H-*pyrazole,
le 3-(2,4-dihydroxyphényl)-4-(4-amino-3-méthylphénoxy)-1*H-*pyrazole,
le 3-(2,4-dihydroxyphényl)-4-(2-amino-5-méthylphénoxy)-1*H-*pyrazole,
le 3-(2,4-dihydroxyphényl)-4-(2-amino-4-méthylphénoxy)-1*H-*pyrazole,
le 3-(2,4-dihydroxyphényl)-4-(3-amino-6-méthylphénoxy)-1*H-*pyrazole,
le 3-(2,4-dihydroxyphényl)-4-(4-amino-3-fluorophénoxy)-1*H-*pyrazole,
le 3-(2,4-dihydroxyphényl)-4-(3,4-difluorophénoxy)-1*H*-pyrazole,
le 3-(2,4-dihydroxyphényl)-4-(2,3-difluorophénoxy)-1*H*-pyrazole,
le 3-(2,4-dihydroxyphényl)-4-(3,5-difluorophénoxy)-1*H*-pyrazole,
le 3-(2,4-dihydroxyphényl)-4-(2,4-difluorophénoxy)-1*H*-pyrazole,
le 3-(2,4-dihydroxyphényl)-4-(2,6-difluorophénoxy)-1*H*-pyrazole,
le 3-(2,4-dihydroxyphényl)-4-(indol-6-yloxy)-1*H*-pyrazole,
le 3-(2,4-dihydroxyphényl)-4-(indol-4-yloxy)-1*H*-pyrazole,
le 3-(2,4-dihydroxyphényl)-4-(indol-5-yloxy)-1*H*-pyrazole,
le 3-(2,4-dihydroxyphényl)-4-(4-cyano-3-fluorophénoxy)-1*H-*pyrazole,
le 3-(2,4-dihydroxyphényl)-4-(2-aminocarbonylphénoxy)-1*H-*pyrazole,
le 3-(2,4-dihydroxyphényl)-4-(4-aminocarbonylphénoxy)-1*H-*pyrazole,
le 3-(2,4-dihydroxyphényl)-4-(4-acétamidophénoxy)-1*H-*pyrazole,
le 3-(2,4-dihydroxyphényl)-4-(2-acétamidophénoxy)-1*H-*pyrazole,
le 3-(2,4-dihydroxyphényl)-4-(3-acétamidophénoxy)-1*H-*pyrazole,
le 3-(2,4-dihydroxyphényl)-4-(3-uréidophénoxy)-1*H*-pyrazole,
le 3-(2,4-dihydroxyphényl)-4-(3-diméthylaminophénoxy)-1*H-*pyrazole,
le 3-(2,4-dihydroxyphényl)-4-(3-méthoxycarbonylphénoxy)-1*H-*pyrazole,
le 3-(2,4-dihydroxyphényl)-4-(2-méthoxycarbonylphénoxy)-1*H-*pyrazole,
le 3-(2,4-dihydroxyphényl)-4-[(4-fluorophényl)sulfanyl]-1*H-*pyrazole,
le 3-(2,4-dihydroxy-5-bromophényl)-4-(3-méthylphénoxy)-1*H-*pyrazole,
le 3-(2,4-dihydroxy-5-bromophényl)-4-(2,3-dibromophénoxy)-1*H-*pyrazole,
le 3-(2,4-dihydroxy-5-bromophényl)-4-(4-fluorophénoxy)-1*H-*pyrazole,
le 3-(2,4-dihydroxy-5-bromophényl)-4-(3-chlorophénoxy)-1*H-*pyrazole,
le 3-(2,4-dihydroxy-5-bromophényl)-4-(4-chlorophénoxy)-1*H-*pyrazole,
le 3-(2,4-dihydroxy-5-bromophényl)-4-(4-éthylphénoxy)-1*H-*pyrazole,
le 3-(2,4-dihydroxy-5-bromophényl)-4-(2,5-diméthylphénoxy)-1 H-pyrazole,
le 3-(2,4-dihydroxy-5-bromophényl)-4-(4-cyanophénoxy)-1*H-*pyrazole,
3-(2,4-diméthoxyphényl)-4-[(4-fluorophényl)sulfanyl]-1*H-*pyrazole,
le 3-(2,4-dihydroxy-5-bromophényl)-4-(3-diméthylamino-phénoxy)-1*H*-pyrazole,
le 3-(2,4-dihydroxy-5-bromophényl)-4-(2,6-difluorophénoxy)-1*H-*pyrazole,
le 3-(2,4-dihydroxy-5-bromophényl)-4-(2,4-difluorophénoxy)-1*H-*pyrazole,
le 3-(2,4-dihydroxy-5-bromophényl)-4-(2-chlorophénoxy)-1*H-*pyrazole,
le 3-(2,4-dihydroxy-5-bromophényl)-4-(2,3-diméthylphénoxy)-1*H*-pyrazole,
le 3-(2,4-dihydroxy-5-bromophényl)-4-(3,5-diméthylphénoxy)-1*H*-pyrazole,
le 3-(2,4-dihydroxy-5-bromophényl)-4-(3-éthylphénoxy)-1*H-*pyrazole,
le 3-(2,4-dihydroxy-5-bromophényl)-4-(2-éthylphénoxy)-1*H-*pyrazole,
le 3-(2,4-dihydroxy-5-bromophényl)-4-(3,4-diméthylphénoxy)-1*H*-pyrazole,
le 3-(2,4-dihydroxy-5-bromophényl)-4-(2,4-diméthylphénoxy)-1*H*-pyrazole,
le 3-(2,4-dihydroxy-5-bromophényl)-4-(2-fluorophénoxy)-1*H-*pyrazole,
le 3-(2,4-dihydroxy-5-bromophényl)-4-(2-méthylphénoxy)-1*H-*pyrazole,
le 3-[2,4-dihydroxy-5-(3-hydroxyméthylpipéridine-1-sulfonyl)-phényl]-4-(2-chlorophénoxy)-1*H*-pyrazole,
le 3-[2,4-dihydroxy-5-diéthylsulfamoylphényl]-4-(2-chloro-4-diéthylsulfamoylphénoxy)-1*H*-pyrazole,
le 3-[2,4-dihydroxy-5-(pyridin-4-ylsulfamoyl)phényl]-4-(2-chloro-phénoxy)-1*H*-pyrazole,
le 3-[2,4-dihydroxy-5-(pyridin-2-ylsulfamoyl)phényl]-4-(2-chloro-phénoxy)-1*H*-pyrazole,
le 3-[2,4-dihydroxy-5-(pyridin-3-ylsulfamoyl)phényl]-4-(2-chloro-phénoxy)-1*H*-pyrazole,
le 3-[2,4-dihydroxy-5-(benzylsulfamoyl)phényl]-4-(2-chloro-phénoxy)-1*H*-pyrazole,
le 3-[2,4-dihydroxy-5-(4-fluorophénylsulfamoyl)phényl]-4-(2-chlorophénoxy)-1*H*-pyrazole,
le 3-[2,4-dihydroxy-5-(3-fluorophénylsulfamoyl)phényl]-4-(2-chlorophénoxy)-1*H*-pyrazole,
le 3-[2,4-dihydroxy-5-(2-fluorophénylsulfamoyl)phényl]-4-(2-chlorophénoxy)-1*H*-pyrazole,
le 3-[2,4-dihydroxy-5-(phénylsulfamoyl)phényl]-4-(2-chloro-phénoxy)-1H-pyrazole,
le 3-[2,4-dihydroxy-5-(2-diéthylaminométhylpipéridine-1-sulfonyl)phényl]-4-(2-chlorophénoxy)-1 H-pyrazole,
le 3-[2,4-dihydroxy-5-(4-méthylpipérazine-1-sulfonyl)phényl]-4-(2-chlorophénoxy)-1*H*-pyrazole,
le 3-[2,4-dihydroxy-5-(pipéridine-1-sulfonyl)phényl]-4-(2-chloro-phénoxy)-1*H*-pyrazole,
le 3-[2,4-dihydroxy-5-(diéthylsulfamoyl)phényl]-4-(2-chloro-phénoxy)-1*H*-pyrazole,
et les sels, solvats, tautomères et stéréoisomères pharmaceutiquement utilisables de ceux-ci, y compris des mélanges de ceux-ci en toutes proportions.

2. Médicaments comprenant au moins un composé selon la revendication 1, choisis dans le groupe constitué par
le 3-(2,4-dihydroxyphényl)-4-(4-fluorophénoxy)-1H-pyrazole,
le 3-(2,4-dihydroxy-5-éthylphényl)-4-(4-fluorophénoxy)-5-méthyl-1 H-pyrazole,
le 3-(2,4-dihydroxyphényl)-4-(4-fluorophénoxy)-5-trifluorométhyl-1 H-pyrazole,
le 3-(2,4-dihydroxyphényl)-4-(4-éthoxyphénoxy)-1*H*-pyrazole,
le 3-(2,4-dihydroxyphényl)-4-phénoxy-1*H*-pyrazole,
le 3-(2,4-dihydroxyphényl)-4-(4-méthylphénoxy)-1*H*-pyrazole,
le 3-(2,4-dihydroxyphényl)-4-(3-méthylphénoxy)-1*H*-pyrazole,
le 3-(2,4-dihydroxyphényl)-4-(2-méthylphénoxy)-1*H*-pyrazole,
le 3-(2,4-dihydroxyphényl)-4-(2-aminophénoxy)-1*H*-pyrazole,
le 3-(2,4-dihydroxyphényl)-4-(3-aminophénoxy)-1*H*-pyrazole,
le 3-(2,4-dihydroxyphényl)-4-(4-aminophénoxy)-1*H*-pyrazole,
le 3-(2,4-dihydroxyphényl)-4-(3-fluorophénoxy)-1*H*-pyrazole,
le 3-(2,4-dihydroxyphényl)-4-(2-fluorophénoxy)-1*H*-pyrazole,
le 3-(2,4-dihydroxyphényl)-4-(4-cyanophénoxy)-1*H*-pyrazole,
le 3-(2,4-dihydroxyphényl)-4-(2-cyanophénoxy)-1*H*-pyrazole,
le 3-(2,4-dihydroxyphényl)-4-(3-cyanophénoxy)-1*H*-pyrazole,
le 3-(2,4-dihydroxyphényl)-4-(2,4-diméthylphénoxy)-1*H-*pyrazole,
le 3-(2,4-dihydroxyphényl)-4-(2,5-diméthylphénoxy)-1*H-*pyrazole,
le 3-(2,4-dihydroxyphényl)-4-(2,6-diméthylphénoxy)-1*H-*pyrazole,
le 3-(2,4-dihydroxyphényl)-4-(3,4-diméthylphénoxy)-1*H-*pyrazole,
le 3-(2,4-dihydroxyphényl)-4-(2-éthylphénoxy)-1*H*-pyrazole,
le 3-(2,4-dihydroxyphényl)-4-(3-éthylphénoxy)-1*H*-pyrazole,
le 3-(2,4-dihydroxyphényl)-4-(3,5-diméthylphénoxy)-1*H-*pyrazole,
le 3-(2,4-dihydroxyphényl)-4-(4-éthylphénoxy)-1*H*-pyrazole,
le 3-(2,4-dihydroxyphényl)-4-(2,3-diméthylphénoxy)-1*H-*pyrazole,
le 3-(2,4-dihydroxyphényl)-4-(4-amino-3-méthylphénoxy)-1*H-*pyrazole,
le 3-(2,4-dihydroxyphényl)-4-(2-amino-5-méthylphénoxy)-1*H-*pyrazole,
le 3-(2,4-dihydroxyphényl)-4-(2-amino-4-méthylphénoxy)-1*H-*pyrazole,
le 3-(2,4-dihydroxyphényl)-4-(3-amino-6-méthylphénoxy)-1*H-*pyrazole,
le 3-(2,4-dihydroxyphényl)-4-(4-amino-3-fluorophénoxy)-1*H-*pyrazole,
le 3-(2,4-dihydroxyphényl)-4-(2-chlorophénoxy)-1*H*-pyrazole,
le 3-(2,4-dihydroxyphényl)-4-(4-chlorophénoxy)-1*H*-pyrazole,
le 3-(2,4-dihydroxyphényl)-4-(3-chlorophénoxy)-1*H*-pyrazole,
le 3-(2,4-dihydroxyphényl)-4-(3,4-difluorophénoxy)-1*H*-pyrazole,
le 3-(2,4-dihydroxyphényl)-4-(2,3-difluorophénoxy)-1*H*-pyrazole,
le 3-(2,4-dihydroxyphényl)-4-(3,5-difluorophénoxy)-1*H*-pyrazole,
le 3-(2,4-dihydroxyphényl)-4-(2,4-difluorophénoxy)-1*H*-pyrazole,
le 3-(2,4-dihydroxyphényl)-4-(2,6-difluorophénoxy)-1*H*-pyrazole,
le 3-(2,4-dihydroxyphényl)-4-(indol-6-yloxy)-1*H*-pyrazole,
le 3-(2,4-dihydroxyphényl)-4-(indol-4-yloxy)-1*H*-pyrazole,
le 3-(2,4-dihydroxyphényl)-4-(indol-5-yloxy)-1*H*-pyrazole,
le 3-(2,4-dihydroxyphényl)-4-(4-cyano-3-fluorophénoxy)-1*H-*pyrazole,
le 3-(2,4-dihydroxyphényl)-4-(2-aminocarbonylphénoxy)-1*H-*pyrazole,
le 3-(2,4-dihydroxyphényl)-4-(4-aminocarbonylphénoxy)-1*H-*pyrazole,
le 3-(2,4-dihydroxyphényl)-4-(4-acétamidophénoxy)-1*H-*pyrazole,
le 3-(2,4-dihydroxyphényl)-4-(2-acétamidophénoxy)-1*H-*pyrazole,
le 3-(2,4-dihydroxyphényl)-4-(3-acétamidophénoxy)-1*H-*pyrazole,
le 3-(2,4-dihydroxyphényl)-4-(3-uréidophénoxy)-1*H*-pyrazole,
le 3-(2,4-dihydroxyphényl)-4-(3-diméthylaminophénoxy)-1*H-*pyrazole,
le 3-(2,4-dihydroxyphényl)-4-(3-méthoxycarbonylphénoxy)-1*H-*pyrazole,
le 3-(2,4-dihydroxyphényl)-4-(2-méthoxycarbonylphénoxy)-1*H-*pyrazole,
le 3-(2,4-dihydroxyphényl)-4-[(4-fluorophényl)sulfanyl]-1*H-*pyrazole,
le 3-(2,4-dihydroxy-5-bromophényl)-4-(3-méthylphénoxy)-1*H-*pyrazole,
le 3-(2,4-dihydroxy-5-bromophényl)-4-(2,3-dibromophénoxy)-1*H-*pyrazole,
le 3-(2,4-dihydroxy-5-bromophényl)-4-(4-fluorophénoxy)-1*H-*pyrazole,
le 3-(2,4-dihydroxy-5-bromophényl)-4-(3-chlorophénoxy)-1*H-*pyrazole,
le 3-(2,4-dihydroxy-5-bromophényl)-4-(4-chlorophénoxy)-1*H-*pyrazole,
le 3-(2,4-dihydroxy-5-bromophényl)-4-(4-éthylphénoxy)-1*H-*pyrazole,
le 3-(2,4-dihydroxy-5-bromophényl)-4-(2,5-diméthylphénoxy)-1*H*-pyrazole,
le 3-(2,4-dihydroxy-5-bromophényl)-4-(4-cyanophénoxy)-1*H-*pyrazole,
3-(2,4-diméthoxyphényl)-4-[(4-fluorophényl)sulfanyl]-1*H-*pyrazole,
le 3-(2,4-dihydroxyphényl)-4-(3-carboxyphénoxy)-1*H*-pyrazole,
le 3-(2,4-dihydroxy-5-bromophényl)-4-(3-diméthylamino-phénoxy)-1*H*-pyrazole,
le 3-(2,4-dihydroxy-5-bromophényl)-4-(2,6-difluorophénoxy)-1*H-*pyrazole,
le 3-(2,4-dihydroxy-5-bromophényl)-4-(2,4-difluorophénoxy)-1*H-*pyrazole,
le 3-(2,4-dihydroxy-5-bromophényl)-4-(2-chlorophénoxy)-1*H-*pyrazole,
le 3-(2,4-dihydroxy-5-bromophényl)-4-(2,3-diméthylphénoxy)-1*H*-pyrazole,
le 3-(2,4-dihydroxy-5-bromophényl)-4-(3,5-diméthylphénoxy)-1*H*-pyrazole,
le 3-(2,4-dihydroxy-5-bromophényl)-4-(3-éthylphénoxy)-1*H-*pyrazole,
le 3-(2,4-dihydroxy-5-bromophényl)-4-(2-éthylphénoxy)-1*H-*pyrazole,
le 3-(2,4-dihydroxy-5-bromophényl)-4-(3,4-diméthylphénoxy)-1*H*-pyrazole,
le 3-(2,4-dihydroxy-5-bromophényl)-4-(2,4-diméthylphénoxy)-1*H*-pyrazole,
le 3-(2,4-dihydroxy-5-bromophényl)-4-(2-fluorophénoxy)-1*H-*pyrazole,
le 3-(2,4-dihydroxy-5-bromophényl)-4-(2-méthylphénoxy)-1*H-*pyrazole,
le 3-[2,4-dihydroxy-5-(3-hydroxyméthylpipéridine-1-sulfonyl)-phényl]-4-(2-chlorophénoxy)-1*H*-pyrazole,
le 3-[2,4-dihydroxy-5-diéthylsulfamoylphényl]-4-(2-chloro-4-diéthylsulfamoylphénoxy)-1*H*-pyrazole,
le 3-[2,4-dihydroxy-5-(pyridin-4-ylsulfamoyl)phényl]-4-(2-chloro-phénoxy)-1*H*-pyrazole,
le 3-[2,4-dihydroxy-5-(pyridin-2-ylsulfamoyl)phényl]-4-(2-chloro-phénoxy)-1*H*-pyrazole,
le 3-[2,4-dihydroxy-5-(pyridin-3-ylsulfamoyl)phényl]-4-(2-chloro-phénoxy)-1*H*-pyrazole,
le 3-[2,4-dihydroxy-5-(benzylsulfamoyl)phényl]-4-(2-chloro-phénoxy)-1*H*-pyrazole,
le 3-[2,4-dihydroxy-5-(4-fluorophénylsulfamoyl)phényl]-4-(2-chlorophénoxy)-1*H*-pyrazole,
le 3-[2,4-dihydroxy-5-(3-fluorophénylsulfamoyl)phényl]-4-(2-chlorophénoxy)-1*H*-pyrazole,
le 3-[2,4-dihydroxy-5-(2-fluorophénylsulfamoyl)phényl]-4-(2-chlorophénoxy)-1*H*-pyrazole,
le 3-[2,4-dihydroxy-5-(phénylsulfamoyl)phényl]-4-(2-chloro-phénoxy)-1*H*-pyrazole,
le 3-[2,4-dihydroxy-5-(2-diéthylaminométhylpipéridine-1-sulfonyl)phényl]-4-(2-chlorophénoxy)-1*H*-pyrazole,
le 3-[2,4-dihydroxy-5-(4-méthylpipérazine-1-sulfonyl)phényl]-4-(2-chlorophénoxy)-1*H*-pyrazole,
le 3-[2,4-dihydroxy-5-(pipéridine-1-sulfonyl)phényl]-4-(2-chloro-phénoxy)-1*H*-pyrazole,
le 3-[2,4-dihydroxy-5-(diéthylsulfamoyl)phényl]-4-(2-chloro-phénoxy)-1*H*-pyrazole,
et les sels, solvats, tautomères et stéréoisomères pharmaceutiquement utilisables de ceux-ci, y compris des mélanges de ceux-ci en toutes proportions.

3. Utilisation de composés selon la revendication 1, et de sels, solvats, tautomères et stéréoisomères pharmaceutiquement utilisables de ceux-ci, y compris des mélanges de ceux-ci en toutes proportions, pour la préparation d'un médicament destiné au traitement et/ou à la prophylaxie de maladies dans lesquelles l'inhibition, la régulation et/ou la modulation de HSP90 joue un rôle.

4. Utilisation selon la revendication 3 de composés selon la revendication 1, et de sels, solvats, tautomères et stéréoisomères pharmaceutiquement utilisables de ceux-ci, y compris des mélanges de ceux-ci en toutes proportions, pour la préparation d'un médicament destiné au traitement ou à la prévention de maladies tumorales, de maladies virales, pour la suppression immunitaire dans le cas des greffes, de maladies induites par des inflammations, de la fibrose kystique, de maladies associées à l'angiogenèse, de maladies infectieuses, de maladies auto-immunes, de l'ischémie, de maladies fibrosantes, pour la promotion de la régénération nerveuse,
pour l'inhibition de la croissance de cancers, de cellules tumorales et de métastases tumorales,
pour la protection de cellules normales contre une toxicité provoquée par une chimiothérapie,
pour le traitement de maladies dans lesquelles un repliement protéique incorrect ou une agrégation constitue un facteur causal principal.

5. Utilisation selon la revendication 4, dans laquelle les maladies tumorales sont le fibrosarcome, le myxosarcome, le liposarcome, le chondrosarcome, le sarcome ostéogénique, le chordome, l'angiosarcome, l'endothéliosarcome, le lymphangiosarcome, le lymphangioendo-théliosarcome, le synoviome, le mésothéliome, le sarcome d'Ewing, le léiomyosarcome, le rhabdomyosarcome, le carcinome du côlon, le cancer du pancréas, le cancer du sein, le cancer de l'ovaire, le cancer de la prostate, le carcinome des cellules squameuses, l'épithélioma basocellulaire, l'adénocarcinome, l'hidradénome, le carcinome des glandes sébacées, le carcinome papillaire, les adénocarcinomes papillaires, les cystadénocarcinomes, le carcinome de la moelle osseuse, le carcinome bronchogénique, le carcinome des cellules rénales, l'hépatome, le carcinome du canal biliaire, le chorio-épithéliome, le séminome, le carcinome embryonnaire, le néphroblastome, le cancer du col de l'utérus, les tumeurs testiculaires, le carcinome du poumon, le carcinome du poumon à petites cellules, le carcinome de la vessie, le carcinome épithélial, le gliome, l'astrocytome, le médulloblastome, le craniopharyngiome, l'épendymome, le pinéalome, l'hémangioblastome, le neurinome de l'acoustique, l'oligodendrogliome, le méningiome, le mélanome, le neuroblastome, le rétinoblastome, la leucémie, le lymphome, le myélome multiple, la macroglobulinémie de Waldenström et la maladie des chaînes lourdes.

6. Utilisation selon la revendication 4, dans laquelle le pathogène viral des maladies virales est choisi dans le groupe constitué par l'hépatite de type A, l'hépatite de type B, l'hépatite de type C, l'influenzavirus, le virus de la varicelle, l'adénovirus, l'herpès simplex de type I (HSV-I), l'herpès simplex de type II (HSV-II), le virus de la peste bovine, le rhinovirus, l'échovirus, le rotavirus, le virus respiratoire syncytial (RSV), le papillomavirus, le papovavirus, le cytomégalovirus, l'échino-virus, l'arbovirus, le hantavirus, le Coxsackie virus, le virus des oreillons, le virus de la rougeole, le virus de la rubéole, le poliovirus, le virus d'immunodéficience humaine de type I (VIH-I) et le virus d'immunodéficience humaine de type II (VIH-II).

7. Utilisation selon la revendication 4, dans laquelle les maladies induites par des inflammations sont la polyarthrite rhumatoïde, l'asthme, la sclérose en plaques, le diabète de type 1, le lupus érythémateux, le psoriasis et l'affection abdominale inflammatoire.

8. Utilisation selon la revendication 4, dans laquelle les maladies associées à l'angiogenèse sont la rétinopathie diabétique, les hémangiomes, l'endométriose et l'angiogenèse tumorale.

9. Utilisation selon la revendication 4, dans laquelle les maladies fibrosantes sont la sclérodermie, la polymyosite, le lupus disséminé, la cirrhose du foie, la formation de kéloïdes, la néphrite interstitielle et la fibrose pulmonaire.

10. Utilisation selon la revendication 4, dans laquelle les maladies dans lesquelles un repliement protéique incorrect ou une agrégation constitue un facteur causal principal sont la tremblante du mouton, la maladie de Creutzfeldt-Jakob, la maladie de Huntington ou la maladie d'Alzheimer.

11. Médicaments comprenant au moins un composé selon la revendication 1 et/ou des sels, solvats, tautomères et stéréoisomères pharmaceutiquement utilisables de celui-ci, y compris des mélanges de ceux-ci en toutes proportions, et au moins un autre composé actif médicamenteux.

12. Ensemble (kit) constitué de conditionnements séparés
(a) d'une quantité efficace d'un composé selon la revendication 1 et/ou de sels, solvats, tautomères et stéréoisomères pharmaceutiquement utilisables de celui-ci, y compris des mélanges de ceux-ci en toutes proportions,
et
(b) d'une quantité efficace d'un autre composé actif médicamenteux.
